(19) Europäisches Patentamt
European Patent Office
Office européen des brevets

(11) **EP 3 181 036 A1**

(12) **EUROPEAN PATENT APPLICATION**

(43) Date of publication:
**21.06.2017 Bulletin 2017/25**

(51) Int Cl.:
*A61B 5/00* (2006.01)     *A61B 5/0205* (2006.01)
*A61B 5/08* (2006.01)     *A61N 1/00* (2006.01)
*A61N 1/04* (2006.01)     *A61N 1/36* (2006.01)

(21) Application number: **15003569.9**

(22) Date of filing: **15.12.2015**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(71) Applicant: **Universität Heidelberg
69117 Heidelberg (DE)**

(72) Inventor: **Nguyen, Xuan Phuc
68163 Mannheim (DE)**

(74) Representative: **Müller-Boré & Partner
Patentanwälte PartG mbB
Friedenheimer Brücke 21
80639 München (DE)**

(54) **MEDICAL DEVICE, METHOD, SYSTEM, CONTROLLER AND COMPUTER PROGRAM PRODUCT**

(57)     A medical device (10) comprising a first circuit (12, C0) for selectively applying power to at least two electrodes applied to a human or animal subject is provided. The first circuit (12, C0) includes: a first switch (S1) electrically connected to a power terminal (PT) and a first electrode terminal (E1), the power terminal (PT) being configured to receive input power from a power source, the first electrode terminal (E1) being configured to be electrically connected to one of said at least two electrodes, the first switch (S1) being arranged in between the power terminal (PT) and the first electrode terminal (E1); a second switch (S2) electrically connected to the power terminal (PT) and a second electrode terminal (E2), the second electrode terminal (E2) being configured to be electrically connected to another one of said at least two electrodes, the second switch (S2) being arranged in between the power terminal (PT) and the second electrode terminal (E2); a third switch (S3) electrically connected to the first electrode terminal (E1) and a ground terminal (GT), the ground terminal (GT) being configured to be electrically connected to a ground, the third switch (S3) being arranged in between the first electrode terminal (E1) and the ground terminal (GT); and a fourth switch (S4) electrically connected to the second electrode terminal (E2) and the ground terminal (GT), the fourth switch (S4) being arranged in between the second electrode terminal (E2) and the ground terminal (GT).

FIG. 2

## Description

The application relates to a medical device, method, system, controller and computer program product for electrical stimulation and/or measurement concerning a human or animal subject.

## BACKGROUND

In medical technology and measurement technology, it is often necessary to record and/or generate electric signals between different electrodes, in other words, to let electric current flow between different electrodes. For example, in medical technology, electric current may be established between two electrodes for electrically stimulating a human or animal subject.

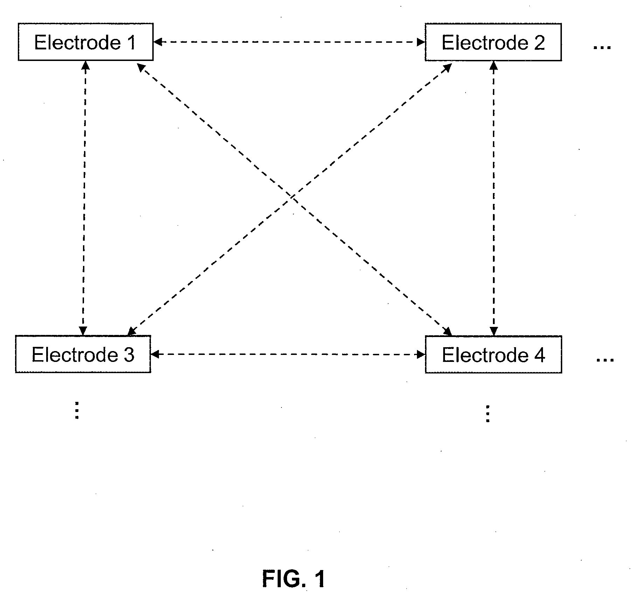

Fig. 1 shows an exemplary arrangement of electrodes that may be employed in medical technology. The exemplary arrangement shown in Fig. 1 includes four electrodes, electrode 1 to electrode 4. The arrangement may, however, include less than or more than four electrodes. Using the exemplary electrodes as shown in Fig. 1, one or more of the following operations may be required:

- applying a specific current pattern signal between any pair of electrodes (e.g. electrodes 1 and 2; electrodes 2 and 3; electrodes 3 and 4 etc. in Fig. 1);

- differentially measuring voltage between any pair of electrodes (e.g. electrodes 1 and 2; electrodes 2 and 3; electrodes 3 and 4 etc. in Fig. 1) for e.g. electromyogram (EMG), electrocardiogram (ECG, EKG), electroencephalography (EEG), electrooculography (EOG), etc.;

- determining a voltage drop during a flow of specific, forced and known current in order to measure impedance between any pair of electrodes (e.g. electrodes 1 and 2; electrodes 2 and 3; electrodes 3 and 4 etc. in Fig. 1) for e.g. electrodermal response (EDR).

## SUMMARY

According to one aspect, a medical device comprising a first circuit for selectively applying power to at least two electrodes applied to a human or animal subject is provided. The first circuit may include the following:

a first switch electrically connected to a power terminal and a first electrode terminal, the power terminal being configured to receive input power from a power source, the first electrode terminal being configured to be electrically connected to one of said at least two electrodes, the first switch being arranged in between the power terminal and the first electrode terminal;
a second switch electrically connected to the power terminal and a second electrode terminal, the second electrode terminal being configured to be electrically connected to another one of said at least two electrodes, the second switch being arranged in between the power terminal and the second electrode terminal;
a third switch electrically connected to the first electrode terminal and a ground terminal, the ground terminal being configured to be electrically connected to a ground, the third switch being arranged in between the first electrode terminal and the ground terminal; and
a fourth switch electrically connected to the second electrode terminal and the ground terminal, the fourth switch being arranged in between the second electrode terminal and the ground terminal.

In the various embodiments and examples described herein, the term "human or animal subject" may be understood as a subject of electrical stimulation and/or measurement using the medical device. Examples of the "human or animal subject" may include, but may not be limited to, a human (e.g. a patient under medical treatment and/or a human under medical testing), an animal (e.g. an animal under medical treatment and/or a laboratory animal), a part of human or animal body and tissue taken from a human or animal.

In some examples, the first circuit may further comprise a ground connecting switch that is electrically connected to the ground terminal and that is configured to electrically connect or disconnect the ground terminal to the ground; and the ground connecting switch may be implemented by two switches electrically connected in parallel to the ground terminal.

In some examples, the medical device may be characterized in that:

the first circuit further comprises a first differential amplifier, a second differential amplifier, a first measurement terminal, a second measurement terminal, a fifth switch, a sixth switch and a resistance;

each of the first measurement terminal and the second measurement terminal is electrically connected to one of two input terminals of the first differential amplifier;

a first end of the third switch is electrically connected to the first electrode terminal and a second end of the third switch is electrically connected to the ground terminal via the fifth switch and the resistance;

the second end of the third switch is further electrically connected to the first measurement terminal;

a first end of the fourth switch is electrically connected to the second electrode terminal and a second end of the fourth switch is electrically connected to the ground terminal via the sixth switch and the resistance;

the second end of the fourth switch is further electrically connected to the second measurement terminal;

a first end of the fifth switch is electrically connected to the second end of the third switch and to the first measurement terminal;

a second end of the fifth switch is electrically connected to a first end of the resistance;

a first end of the sixth switch is electrically connected to the second end of the fourth switch and to the second measurement terminal;

a second end of the sixth switch is electrically connected to the first end of the resistance;

a second end of the resistance is electrically connected to the ground terminal; and

each of the first and second ends of the resistance is electrically connected to one of two input terminals of the second differential amplifier.

In some other examples, the medical device may be characterized in that:

the first circuit further comprises a first differential amplifier, a second differential amplifier, a circuit breaker, a fifth switch, a sixth switch and a resistance;

each of the first and second electrode terminals is electrically connected to one of two input terminals of the first differential amplifier via the circuit breaker, the circuit breaker being configured to electrically disconnect the first differential amplifier from the first and second electrode terminals in case a voltage applied between the first and second electrode terminals is higher than a specified threshold value;

a first end of the third switch is electrically connected to the first electrode terminal and a second end of the third switch is electrically connected to the ground terminal via the fifth switch and the resistance;

a first end of the fourth switch is electrically connected to the second electrode terminal and a second end of the fourth switch is electrically connected to the ground terminal via the sixth switch and the resistance;

the second end of the third switch and the second end of the fourth switch are electrically connected to each other;

a first end of the fifth switch is electrically connected to the second end of the third switch and a second end of the fifth switch is electrically connected to a first end of the resistance;

a first end of the sixth switch is electrically connected to the second end of the fourth switch and a second end of the sixth switch is electrically connected to the first end of the resistance; '

a second end of the resistance is electrically connected to the ground terminal; and

each of the first and second ends of the resistance is electrically connected to one of two input terminals of the second differential amplifier.

In the various embodiments and examples described herein, the "specified threshold value" may be understood as indicating a predetermined or predeterminable threshold value.

The medical device may further comprise at least one second circuit, wherein:

said at least one second circuit has the features of the first circuit;

the first and second electrode terminals of said at least one second circuit are configured to be electrically connected to electrodes that are different from the electrodes to be connected to the first and second electrode terminals of the first circuit;

the first circuit and said at least one second circuit may be configured to receive input power from different power sources; and

the first circuit and said at least one second circuit may be galvanically isolated from one another, either by true galvanic isolation or virtual galvanic isolation.

According to another aspect, a method performed by a controller configured to control the medical device is provided. The method may comprise controlling the medical device to operate, in accordance with a setting made by a user, in one of the following modes:

a stimulation mode in which a part of the human or animal subject is electrically stimulated by two electrodes connected to the first and second electrode terminals, wherein the controller controls the medical device to set:

the first and fourth switches to a closed state and the second and third switches to an open state; or

the first and fourth switches to the open state and the second and third switches to the closed state;

an inactive mode wherein the controller controls the medical device to set the first, second, third and fourth switches to the open state;

a short circuit mode wherein the controller controls the medical device to set the first and second switches to the open state and the third and fourth switches to the closed state.

In the various embodiments and examples disclosed herein, the "closed state" of a switch may refer to a state where the switch is made conductive. In the "closed state" of a switch, current may flow from one end of the switch to the other end of the switch. Further, in the various embodiments and examples disclosed herein, the "open state" of a switch may refer to a state where the switch is made substantially non-conductive. In the "open state", the switch may block current so that no current or only a negligible amount of current can flow from one end of the switch to the other end of the switch.

In the method, when the controller is configured to control the medical device including the first circuit that further comprises the ground connecting switch,

in the stimulation mode, the controller may control the medical device to set the ground connecting switch to the closed state; and

in the inactive mode and the short circuit mode, the controller controls the medical device to set the ground connecting switch to the open state.

In the method, when the controller is configured to control the medical device including the first circuit that further comprises the first and second differential amplifiers, the first and second measurement terminals, the fifth and sixth switches and the resistance,

in the inactive mode, the controller may control the medical device to set the fifth and sixth switches to the closed state while setting the first, second, third and fourth switches to the open state;

in the stimulation mode, the controller may control the medical device to set the fifth and sixth switches to the closed state;

in the short circuit mode, the controller controls the medical device to set the first and second switches to the open state and the third, fourth, fifth and sixth switches to the closed state;

the controller may be further configured to control the medical device to operate, in accordance with the setting by the user, in the stimulation mode, the inactive mode, the short circuit mode, a signal measurement mode or an impedance measurement mode, wherein

in the signal measurement mode, the controller may control the medical device to:

set the first, second, fifth and sixth switches to the open state and the third and fourth switches to the closed state; and

determine a signal between the first and second electrode terminals using an output from the first differential amplifier; and

in the impedance measurement mode, the controller controls the medical device to:

set the third and fourth switches to the closed state while setting:

the first and sixth switches to the closed state and the second and fifth switches to the open state; or

the first and sixth switches to the open state and the second and fifth switches to the closed state; and

determine an impedance between the first and second electrode terminals using outputs from the first and second differential amplifiers.

In the method, when the controller is configured to control the medical device including the first circuit that further comprises the first and second differential amplifiers, the circuit breaker, the fifth and sixth switches and the resistance,

in the stimulation mode, the controller may control the medical device to:

set one or both of the fifth and sixth switches to the closed state; and

set the circuit breaker (CB) to the open state;

in the inactive mode, the controller may control the medical device to set the fifth and sixth switches to the closed state and the circuit breaker (CB) to the open state;

in the short circuit mode, the controller (20) controls the medical device (10) to set the fifth and sixth switches (S5, S6) to the open state and the circuit breaker (CB) to the open state; and

the controller may be configured to control the medical device to operate, in accordance with the setting by the user, in the stimulation mode, the inactive mode, the short circuit mode or a signal measurement mode, wherein, in the signal measurement mode, the controller may control the medical device to:

set the first, second, third, fourth, fifth and sixth switches to the open state;
set the circuit breaker to the closed state; and
determine a signal between the first and second electrode terminals using an output from the first differential amplifier.

According to yet another aspect, a method performed by a controller configured to control the medical device that comprises said at least one second circuit is provided. The method may comprise:

controlling the medical device to operate in a stimulation mode in case of electrically stimulating a part of the human or animal subject by a pair of electrodes connected to a pair of electrode terminals selected from the first and second electrode terminals in the first and second circuits, wherein, in the stimulation mode, the controller controls the medical device to set each switch in the first and second circuits to the closed state or the open state so as to:

electrically connect one of the selected pair of electrode terminals to the power terminal of the circuit comprising the one of the selected pair of electrode terminals;
electrically connect another one of the selected pair of electrode terminals to the ground terminal of the circuit comprising the other one of the selected pair of electrode terminals; and
electrically disconnect the first and second electrode terminals other than the selected pair of electrode terminals from the ground terminal and from the power terminal.

In the method performed by a controller configured to control the medical device that comprises said at least one second circuit, where each of the first circuit and said at least one second circuit comprises the first and second differential amplifiers, the first and second measurement terminals, the fifth and sixth switches and the resistance,
in the stimulation mode, the controller may control the medical device to set the fifth and sixth switches of the first and second circuits to the closed state;
the controller may be further configured to control the medical device to operate in an impedance measurement mode in case of measuring an impedance between a pair of electrode terminals selected from the first and second electrode terminals in the first and second circuits, wherein, in the impedance measurement mode, the controller may control the medical device to:

when the selected pair of electrode terminals are comprised in a same circuit,

set the third and fourth switches of that circuit to the closed state while setting:

the first and sixth switches of that circuit to the closed state and the second and fifth switches of that circuit to the open state; or
the first and sixth switches of that circuit to the open state and the second and fifth switches of that circuit to the closed state; and

set the first and second switches of the first or second circuit that does not comprise the selected pair of electrode terminals to the open state; and

when each one of the selected pair of electrode terminals are comprised in a different circuit, set each switch of the first and second circuits to the closed state or the open state so as to:

electrically connect one of the selected pair of electrode terminals to the power terminal and the first or second measurement terminal of the circuit comprising the one of the selected pair of electrode terminals;
electrically connect, to the ground terminal via the resistance, the first or second measurement terminal that is comprised in the circuit comprising the one of the selected pair of electrode terminals and that is not electrically connected to the one of the selected pair of electrode terminals;
electrically connect another one of the selected pair of electrode terminals to the first or second measurement terminal of the circuit comprising the other one of the selected pair of electrode terminals;
electrically connect, to the ground terminal via the resistance, the first and second measurement terminals of the circuit comprising the other one of the selected pair of electrode terminals; and
electrically disconnect the first and second electrode terminals other than the selected pair of electrode terminals from the ground terminal and from the power terminal.

According to yet another aspect, a computer program product, particularly embodied tangibly on a computer-readable storage medium and/or embodied as a signal or data stream, is provided. The computer program product may comprise computer-readable instructions that, when loaded and run on a suitable system or computer, cause the system or computer to perform the method according to any one of the above-stated aspects.

According to yet another aspect, a controller is provided. The controller may be configured to perform the method according to any one of the above-stated aspects.

According to yet another aspect, a system is provided. The system may comprise the medical device and the controller. The system may further comprise a floating current source as the power source that supplies the input power to the power terminal of the medical device.

According to a further aspect, there is provided a medical device, in particular according to the above aspect or a particular embodiment thereof, for influencing a breathing condition of a patient (i.e. of a human or animal subject), comprising: at least one electrode patch to be externally applied to the patient, wherein the electrode patch comprises a plurality of electrodes, and a control unit, which is to be signal connected to the electrode patch and which comprises a microcontroller operable to apply at least one specified stimulus to the patient via the electrodes of the electrode patch in case an abnormal breathing condition of the patient is determined or predicted.

Particularly, the control unit may monitor and analyze one or more signals received from the electrodes of the electrode patch to detect or predict the abnormal breathing condition of the patient.

Further particularly, the control unit may comprise a database and analyses the one or more signals received from the electrodes of the electrode patch on the basis of reference data stored in the database.

Further particularly, the electrode patch may comprise at least one microphone and/or at least one vibration sensing device for sensing noises and/or vibrations generated by the patient and is able to transmit signals reflecting the sensed noises and/or vibrations to the control unit.

Further particularly, the electrode patch may be configured as an adhesive patch which can be placed and secured to the patient by means of an adhesive layer provided thereon and/or wherein the electrode patch comprises one or more securing members to fixedly secure the electrode patch to the patient in a specified position.

Further particularly, the electrode patch may be configured such as to be arrangeable on the patient in proximity and below of a chin of the patient such that at least one pair of electrodes are arranged adjacent to an arteria carotis of the patient.

Further particularly, the control unit may comprises a first circuit for selectively applying power to the at least two electrodes, the first circuit including: a first switch electrically connected to a power terminal and a first electrode terminal, the power terminal being configured to receive input power from a power source, the first electrode terminal being configured to be electrically connected to one of said at least two electrodes, the first switch being arranged in between the power terminal and the first electrode terminal; a second switch electrically connected to the power terminal and a second electrode terminal, the second electrode terminal being configured to be electrically connected to another one of said at least two electrodes, the second switch being arranged in between the power terminal and the second electrode terminal; a third switch electrically connected to the first electrode terminal and a ground terminal, the ground terminal being configured to be electrically connected to a ground, the third switch being arranged in between the first electrode terminal and the ground terminal; and a fourth switch electrically connected to the second electrode terminal and the ground terminal, the fourth switch being arranged in between the second electrode terminal and the ground terminal.

Further particularly, the medical device may further comprise at least one second circuit, wherein: said at least one second circuit may have the features of the first circuit; the first and second electrode terminals of said at least one second circuit may be configured to be electrically connected to electrodes that are different from the electrodes to be connected to the first and second electrode terminals of the first circuit; the first circuit and said at least one second circuit may be configured to receive input power from different power sources; and the first circuit and said at least one second circuit may be galvanically isolated from one another.

According to a further aspect of the invention, there is provided a method performed by a controller configured to control the medical device according to any one of the preceding claims, wherein the method comprises the following steps: receiving by a control unit of one or more signals from a plurality of electrodes of at least one electrode patch externally applied to the patient; analyzing the one or more signals by means of a controller of the control unit to detect or predict an abnormal breathing condition of the patient ; and applying at least one specified stimulus to the patient via the electrodes of the electrode patch in case an abnormal breathing condition of the patient is determined or predicted.

Further particularly, the control unit periodically or continuously may monitor and analyse one or more signals received from the electrodes of the electrode patch to detect or predict the abnormal breathing condition of the patient.

Further particularly, the one or more signals received from the electrodes of the electrode patch may be analyzed by the control unit on the basis of reference data retrieved from a database.

Further particularly, the controller of the control unit may detect or determine or predict an abnormal breathing condition of the patient based on one or more signals received from at least one microphone and/or at least one vibration sensing device for sensing noises and/or vibrations generated by the patient.

Further particularly, the method may further comprise steps of: placing and securing to the patient the electrode patch in a specified position to the patient by means of an adhesive layer provided on the electrode patch and/or by means of one or more securing members of the electrode patch.

Further particularly, the method may comprise controlling the medical device to operate, in accordance with a setting made by a user, in one of the following modes: (a) a stimulation mode in which a part of the human or animal subject is electrically stimulated by two electrodes connected to the first and second electrode terminals, wherein the controller controls the medical device to set: (i) the first and fourth switches to a closed state and the second and third switches to an open state; or (ii) the first and fourth switches to the open state and the second and third switches to the closed state; (b) an inactive mode wherein the controller controls the medical device to set the first, second, third and fourth switches to the open state; (c) a short circuit mode wherein the controller controls the medical device to set the first and second switches to the open state and the third and fourth switches to the closed state.

According to another aspect, there is provided a computer program product, particularly embodied tangibly on a computer-readable storage medium and/or embodied as a signal or data stream, comprising computer-readable instructions that, when loaded and run or executed on a suitable system comprising a computer, cause the computer to perform the method according to the above aspect of the invention or a particular embodiment thereof.

According to a further aspect, there is provided a method of influencing a breathing condition of a patient, comprising the following steps: externally applying at least one electrode patch comprising a plurality of electrodes to the patient; (particularly periodically or substantially constantly) monitoring one or more signals sensed by the electrodes on the electrode patch in view of determining or predicting an abnormal breathing condition of the patient (such as apnea or hypopnea); and applying at least one specified stimulus to the patient via the electrodes of the electrode patch in case the abnormal breathing condition of the patient is determined or predicted.

The subject matter described in the application can be implemented as a method or as a system, possibly in the form of one or more computer program products. The subject matter described in the application can be implemented in a data signal or on a machine readable medium, where the medium is embodied in one or more information carriers, such as a CD-ROM, a DVD-ROM, a semiconductor memory, or a hard disk. Such computer program products may cause a data processing apparatus to perform one or more operations described in the application.

In addition, subject matter described in the application can also be implemented as a system including a processor, and a memory coupled to the processor. The memory may encode one or more programs to cause the processor to perform one or more of the methods described in the application. Further subject matter described in the application can be implemented using various machines.

## BRIEF DESCRIPTION OF THE DRAWINGS

Details of exemplary embodiments are set forth below with reference to the exemplary drawings. Other features will be apparent from the description, the drawings, and from the claims. It should be understood, however, that even though embodiments are separately described, single features of different embodiments may be combined to further embodiments.

**Fig. 1** shows an exemplary arrangement of electrodes that may be employed in medical technology.

**Fig. 2** shows an exemplary functional block diagram of a system including a medical device according to the embodiments described herein.

**Fig. 3** shows an example of the circuit 12 that may be included in the medical device 10 according to Embodiment 1.

**Fig. 4** shows a part of an exemplary configuration of the medical device 10 that includes more than one circuits 12 according to Embodiment 1.

**Fig. 5** shows a part of another exemplary configuration of the medical device 10 that includes more than one circuits 12 according to Embodiment 1.

**Fig. 6** shows examples of a monophasic pulse signal and a biphasic pulse signal.

**Fig. 7** shows exemplary signal patterns of the current signals that may be applied to the circuit(s) 12 of the medical device 10 in case of LF (low frequency) stimulation.

**Fig. 8** shows exemplary signal patterns of the current signals that may be applied to the circuit(s) 12 of the medical device 10 in case of MF (middle frequency) stimulation.

**Fig. 9** shows yet another exemplary signal pattern of the current signal that may be applied to the circuit 12 of the medical device 10 in case of MF stimulation.

**Figs. 10 to 12** show exemplary paths of current during the stimulation mode of the medical device 10 having the circuits 12 as shown in Fig. 5.

**Fig. 13** shows an example of current paths when stimulation is performed using the medical device 10.

**Fig. 14** shows an example of the circuit 12 that may be included in the medical device 10 according to Embodiment 2.

**Fig. 15** shows another example of the circuit 12 that may be included in the medical device 10 according to Embodiment 2.

**Fig. 16** shows a part of exemplary configuration of the medical device 10 that includes more than one circuits 12 according to Embodiment 2.

**Fig. 17** shows a part of another exemplary configuration of the medical device 10 that includes more than one circuits 12 according to Embodiment 2.

**Fig. 18** shows exemplary signal patterns in case of LF stimulation using the medical device 10 including one or more circuit 12 having a ground connecting switch according to Embodiment 2.

**Fig. 19** shows exemplary signal patterns in case of MF stimulation using the medical device 10 including one or more circuit 12 having a ground connecting switch according to Embodiment 2.

**Fig. 20** shows an example of the circuit 12 that may be included in the medical device 10 according to Embodiment 3.

**Fig. 21** shows another example of the circuit 12 that may be included in the medical device 10 according to Embodiment 3.

**Fig. 22** shows an example of a simplified circuit when the circuit shown in Fig. 21 is used for impedance measurement.

**Fig. 23** shows a part of an exemplary configuration of the medical device 10 that includes more than one circuits 12 according to Embodiment 3.

**Fig. 24** shows a part of another exemplary configuration of the medical device 10 that includes more than one circuits 12 according to Embodiment 3. Here a voltage source is used instead of a current source.

**Fig. 25A** shows yet another example of the circuit 12 that may be included in the medical device 10 according to Embodiment 3.

**Fig. 25B** shows a part of yet another exemplary configuration of the medical device 10 that includes more than one circuits 12 according to Embodiment 3.

**Figs. 26A, 26B, 27A and 27B** show exemplary paths of current during the stimulation mode of the medical device 10 having the circuits 12 as shown in Fig. 23 or 25B.

**Figs. 28A and 28B** show exemplary paths of current during the impedance measurement mode of the medical device 10 having the circuits 12 as shown in Fig. 23 and 25B, respectively.

**Fig. 29** shows exemplary signal patterns in case of LF stimulation using the medical device 10 including the circuits 12 with true galvanic isolation.

**Fig. 30** shows exemplary signal patterns in case of MF stimulation using the medical device 10 including the circuits 12 with true galvanic isolation.

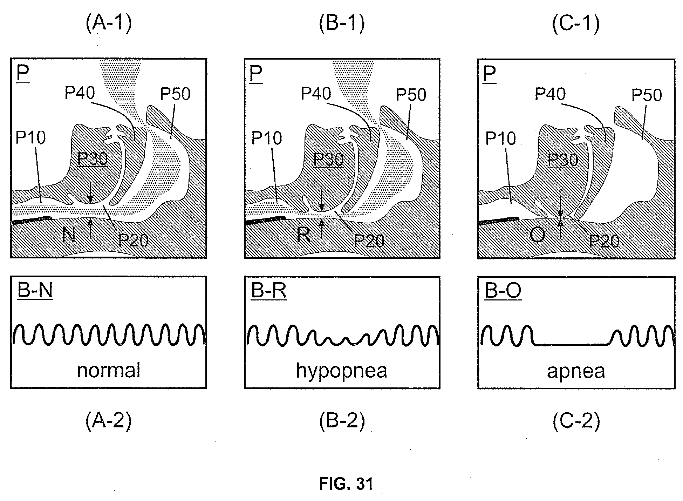

**Fig. 31** shows different breathing situations of a patient, wherein **Fig. 31 A-1** shows a normal breathing situation where respiratory ducts of a patient are open and **Fig. 31A-2** shows an airflow of the patient corresponding to the normal breathing situation; **Fig. 31 B-1** shows a restricted breathing situation where respiratory ducts of a patient are restricted and **Fig. 31 B-2** shows an airflow of the patient corresponding to the restricted breathing situation; and **Fig. 31 C-1** shows an obstructed breathing situation where respiratory ducts of a patient are obstructed and **Fig. 31C-2** shows an airflow of the patient corresponding to the obstructed breathing situation.

**Fig. 32** shows a basic setup of an embodiment of a medical device 100 for influencing the breathing of a patient P.

**Fig. 33** shows an arrangement of at least one electrode patch on a patient according to an embodiment of the invention.

**Fig. 34** shows an arrangement of at least one electrode patch on a patient according to a further embodiment of the invention.

**Fig. 35 (A) to (D)** shows exemplary signal patterns reflecting frequency dependent resistance measurements between different pairs of electrodes particularly arranged according to Fig. 34.

**Fig. 36** shows an exemplary implementation of the circuit C0 and a possible implementation of the signal current source CS0 as a floating current source in accordance with Fig. 15.

**Fig. 37** shows an exemplary signal pattern in case of MF stimulation using the circuit(s) 12 with the ground connecting switch(es) SG and the floating current source CSO as shown in Fig. 36.

**Fig. 38 (A) and (B)** shows the possible end positions of the tongue - free and closed airways - as well as the arrangement of measurement and stimulation electrodes on a patient. **Fig. 38 (A)** shows an obstructed breathing situation where respiratory ducts (or an airway) of the patient are obstructed and **Fig. 38 (B)** shows a normal breathing situation where respiratory ducts (or an airway) of the patient are open.

**Fig. 39** schematically shows different muscles and nerves in an area of a throat and chin of a patient.

## DETAILED DESCRIPTION OF EMBODIMENTS

In the following text, a detailed description of examples will be given with reference to the drawings. It should be understood that various modifications to the examples may be made. In particular, one or more elements of one example may be combined and used in other examples to form new examples.

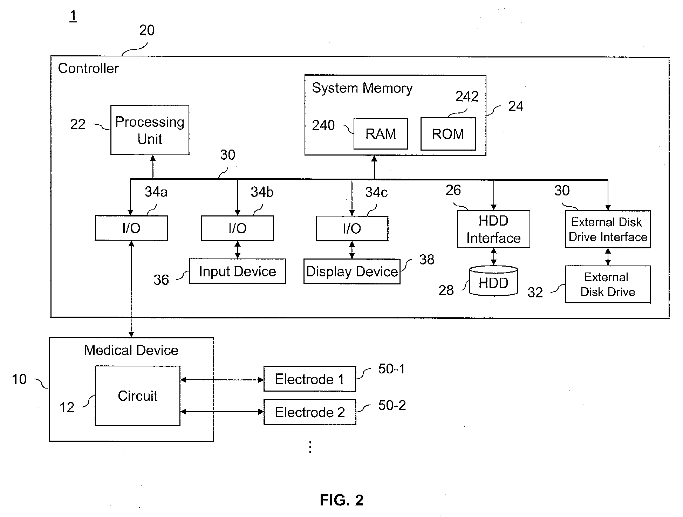

**Fig. 2** shows an exemplary functional block diagram of a system including a medical device according to the embodiments described herein. An exemplary system 1 shown in Fig. 2 may include a medical device 10 and a controller 20.

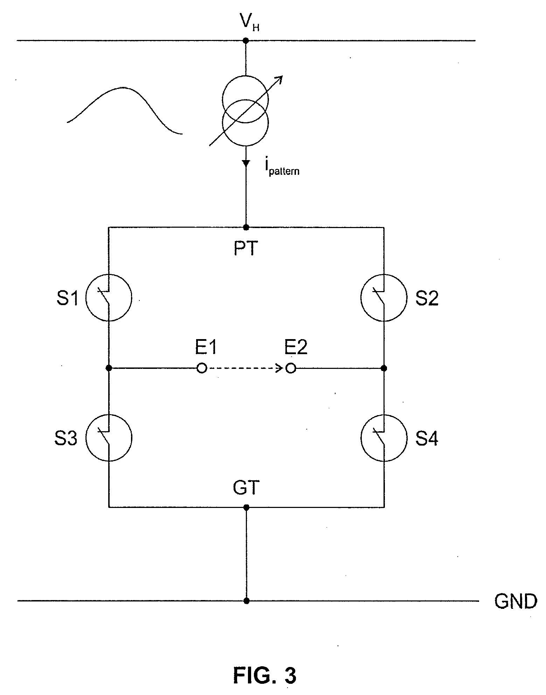

The medical device 10 may comprise one or more circuit 12 for selectively applying power to at least two electrodes 50-1, 50-2 applied to a human or animal subject. The circuit 12 of the medical device 10 may be electrically connected to the at least two electrodes 50-1, 50-2. The at least two electrodes 50-1, 50-2 may be, for example, placed on different parts of a human or animal subject. In an example, each of the at least two electrodes 50-1, 50-2 may be provided on a clip and the clip may hold a part of a human or animal subject so that the electrode contacts the part of a human or animal subject. The details of the circuit 12 will be described below, referring to Figs. 3 to 30.

It should be noted that, although Fig. 2 shows only one circuit 12 comprised in the medical device 10, the medical device 10 may comprise more than one circuits 12. Similarly, although Fig. 2 shows only two electrodes 50-1, 50-2, the medical device 10 may be configured to be connectable to more than two electrodes 50.

The controller 20 may be configured to control the medical device 10. The controller 20 may comprise a processing unit 22, a system memory 24, hard disk drive (HDD) interface 26, external disk drive interface 30, and input/output (I/O) interfaces 34. These components of the controller 20 are coupled to each other via a system bus 30. The processing unit 22 may perform arithmetic, logic and/or control operations by accessing the system memory 24. The system memory 24 may store information and/or instructions for use in combination with the processing unit 22. The system memory 24 may include volatile and non-volatile memory, such as a random access memory (RAM) 240 and a read only memory (ROM) 242. A basic input/output system (BIOS) containing the basic routines that helps to transfer information between elements within the general purpose computer, such as during start-up, may be stored in the ROM 242. The system bus 30 may be any of several types of bus structures including a memory bus or memory controller, a peripheral bus, and a local bus using any of a variety of bus architectures.

The controller 20 shown in Fig. 2 may include a hard disk drive (HDD) 28 for reading from and writing to a hard disk (not shown), and an external disk drive 32 for reading from or writing to a removable disk (not shown). The removable disk may be a magnetic disk for a magnetic disk drive or an optical disk such as a CD ROM for an optical disk drive. The HDD 28 and the external disk drive 32 are connected to the system bus 30 by a HDD interface 26 and an external disk drive interface 30, respectively. The drives and their associated computer-readable media provide non-volatile storage of computer-readable instructions, data structures, program modules and other data for the general purpose computer. The data structures may include relevant data for the implementation of the method for controlling the medical device 10, as described herein. The relevant data may be organized in a database, for example a relational or object database.

Although the exemplary environment described herein employs a hard disk (not shown) and an external disk (not shown), it should be appreciated by those skilled in the art that other types of computer readable media which can store data that is accessible by a computer, such as magnetic cassettes, flash memory cards, digital video disks, random access memories, read only memories, and the like, may also be used in the exemplary operating environment.

A number of program modules may be stored on the hard disk, external disk, ROM 242 or RAM 240, including an operating system (not shown), one or more application programs (not shown), other program modules (not shown), and program data (not shown). The application programs may include at least a part of the functionality as will be described below.

The controller 20 may be connected to the medical device 10 for sending control signals to the medical device 10 and receiving signals from medical device 10. The medical device 10 may be connected to the system bus 30 of the controller 20 via I/O interface 34a.

The controller 20 shown in Fig. 2 may also include an input device 36 such as mouse and/or keyboard, and display device 38, such as liquid crystal display. The input device 36 and the display device 38 are connected to the system bus 30 via I/O interfaces 34b, 34c.

It should be noted that the above-described controller 20 employing a general purpose computer is only one example of an implementation of the exemplary embodiments described herein. For example, the controller 20 may include additional components not shown in Fig. 2, such as network interfaces for communicating with other devices and/or computers.

In addition or as an alternative to an implementation using a general purpose computer as shown in Fig. 2, a part or all of the functionality of the exemplary embodiments described herein may be implemented as one or more hardware circuits. Examples of such hardware circuits may include but are not limited to: Large Scale Integration (LSI), Application Specific Integrated Circuit (ASIC) and Field Programmable Gate Array (FPGA).

In some examples, the medical device 10 and the controller 20 shown in Fig. 2 may be implemented as two different apparatuses. In other examples, the medical device 10 and the controller 20 may be implemented as a single apparatus that comprises all the functions of the medical device 10 and the controller 20.

The following describes exemplary embodiments of the circuit 12 and their exemplary applications.

### Embodiment 1: Circuit with Four Switches

< Basic Circuit Configuration and Switch Control >

**Fig. 3** shows an example of the circuit 12 that may be included in the medical device 10 according to Embodiment 1. The exemplary circuit shown in Fig. 3 may include four switches S1, S2, S3 and S4, a power terminal PT, a first electrode terminal E1, a second electrode terminal E2 and a ground terminal GT.

Each of the four switches S1 to S4 may be configured to set its state according to a control signal received from the controller 20. The control signal may indicate whether the switch should be set to an open state or a closed state. In the open state, the switch may be substantially non-conductive. Accordingly, when the switch is in the open state, no current or only a negligible amount of current can flow from one end of the switch to the other end of the switch. In the closed state, the switch may be made conductive. Accordingly, when the switch is in the closed state, current may flow from one end of the switch to the other end of the switch. Each of the four switches S1 to S4 may be implemented by transistors, for example.

The power terminal PT may be configured to receive input power from a power source. The first electrode terminal E1 may be configured to be electrically connected to one of the at least two electrodes 50-1, 50-2. The second electrode terminal E2 may be configured to be electrically connected to another one of the at least two electrodes 50-1, 50-2. The ground terminal GT may be configured to be electrically connected to a ground.

The first switch S1 may be electrically connected to the power terminal PT and the first electrode terminal E1. The first switch S1 may be arranged in between the power terminal PT and the first electrode terminal E1. The second switch S2 may be electrically connected to the power terminal PT and the second electrode terminal E2. The second switch S2 may be arranged in between the power terminal PT and the second electrode terminal E2. The third switch S3 may be electrically connected to the first electrode terminal E1 and the ground terminal GT. The third switch S3 may be arranged in between the first electrode terminal E1 and the ground terminal GT. The fourth switch S4 may be electrically connected to the second electrode terminal E2 and the ground terminal GT. The fourth switch S4 may be arranged in between the second electrode terminal E2 and the ground terminal GT.

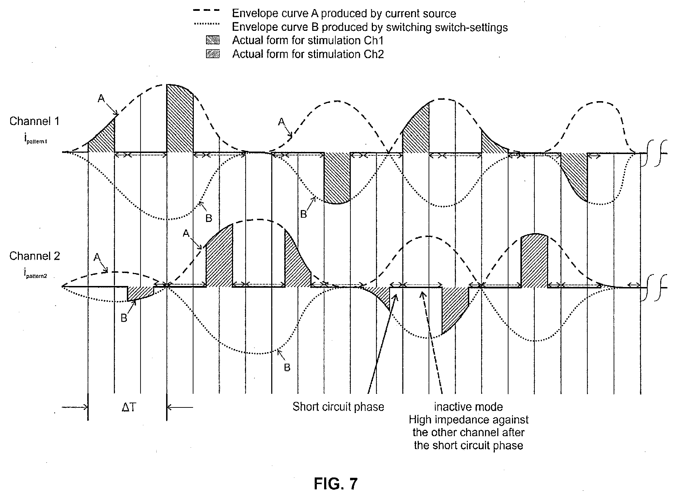

The power source may be, for example, a current source that provides a current signal $i_{pattern}$ that defines the envelope curve or modulation of a stimulation current signal for electrically stimulating a human or animal subject by applying power to the electrodes connected to the electrode terminals E1 and E2. The current signal $i_{pattern}$ may comprise either a direct current (DC) or an alternating current (AC). The actual stimulation current signal that flows from one electrode to the other electrode may comprise, however, the "sampled" current signal $i_{pattern}$. The sampling of the current signal $i_{pattern}$ may be made by controlling the states of the switches S1, S2, S3 and S4, which will be explained more in detail below with reference to Figs. 7 to 9.

In case the medical device 10 includes a circuit with the exemplary configuration as shown in Fig. 3 as the circuit 12, the controller 20 may control the medical device 10 to operate, in accordance with a setting made by a user, in one of the exemplary modes shown in Table 1.

Table 1: Exemplary Operation Modes for the Circuit shown in Fig. 3

| Mode | Switch Settings | Function |
|---|---|---|
| Stimulation (E1 → E2) | S1 & S4 closed; S2 & S3 open | Sampled current $i_{pattern}$ flows from E1 to E2 and the ground as stimulation current. |
| Stimulation (E2 → E1) | S2 & S3 closed; S1 & S4 open | Sampled current $i_{pattern}$ flows from E2 to E1 and the ground as stimulation current. |
| Inactive | S1 to S4 open | No current flow into and out of the circuit possible. |
| Short Circuit | S1 & S2 open; S3 & S4 closed | Short circuit phase. |

As can be seen from Table 1, during the stimulation modes, a pair of switches S1, S4 or the other pair of switches S2, S3 is set to a closed state and the remaining pair of switches is set to an open state. During the stimulation mode E1 → E2, current may flow from the power source through the power terminal PT, the first switch S1, the first electrode terminal E1, the second electrode terminal E2 and the fourth switch S4, the ground terminal GT and to the ground. During the stimulation mode E2 → E1, current may flow from the power source through the power terminal PT, the second switch S2, the second electrode terminal E2, the first electrode terminal E1, the third switch S3, the ground terminal GT and to the ground. The stimulation modes enable electrical stimulation of a part of a human or animal subject by two electrodes connected to the first and second electrode terminals E1, E2.

According to Table 1, during the inactive mode, all of the switches S1, S2, S3 and S4 are set to the open state and no current can flow into or out of the circuit.

Further according to Table 1, during the short circuit mode, the first and second switches S1, S2 are set to the open state and the third and fourth switches S3, S4 are set to the closed state. During the short circuit mode, the circuit may be electrically disconnected from the power source and the first and second electrode terminals E1, E2 may be electrically connected to the ground.

< Possible Extensions of the Circuit >

As stated above with reference to Fig. 2, the medical device 10 may comprise more than one circuits 12 and may be connected to more than two electrodes 50.

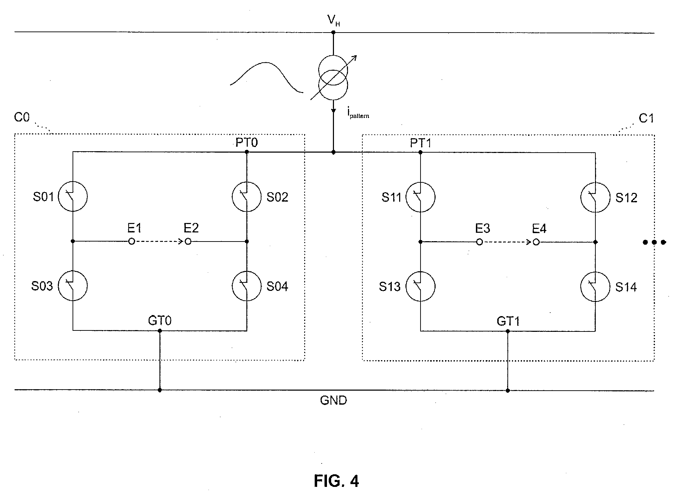

Fig. 4 shows a part of an exemplary configuration of the medical device 10 that includes more than one circuits 12 according to the present Embodiment 1.

As shown in Fig. 4, the medical device 10 may include a first circuit C0 and at least one second circuit C1. The first circuit C0 and the at least one second circuit C1 may be connected in parallel. Each of the first circuit C0 and the at least one second circuit C1 shown in Fig. 4 has the exemplary circuit configuration as shown in Fig. 3. The first and second electrode terminals E3, E4 of the at least one second circuit C1 may be electrically connected to electrodes 50 that are different from the electrodes to be connected to the first and second electrode terminals E1, E2 of the first circuit C0. The first circuit C0 and the at least one second circuit C1 may be configured to receive input power from the same power source. For example, in Fig. 4, the power terminals PT0, PT1 of the first circuit C0 and of the at least one second circuit C1 are connected to a single current source.

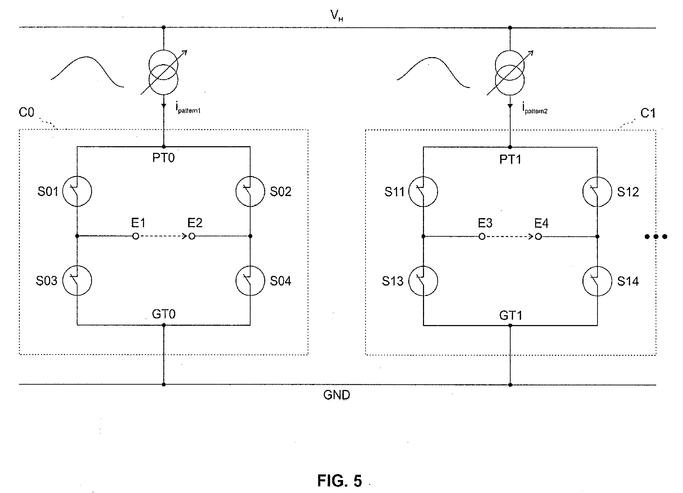

Fig. 5 shows a part of another exemplary configuration of the medical device 10 that includes more than one circuits 12 according to the present Embodiment 1.

In the example of Fig. 5, the medical device 10 has a configuration identical to that shown in Fig. 4 except that the first circuit C0 and the at least one second circuit C1 are configured to receive input power from different power sources. For instance, in Fig. 5, the power terminals PT0, PT1 of the first circuit C0 and of the at least one second circuit C1 are connected to different current sources. The current sources shown in Fig. 5 may be independent of each other. Accordingly, different envelope current signals can be sampled and applied to different pairs of electrodes depending on which circuit that pair of electrodes is connected to.

When using different power sources for more than one circuits 12, galvanic isolation of the circuits receiving power from different power sources must be considered. Galvanic isolation is a principle of isolating functional sections of electrical systems to current flow. In medical technology, galvanic isolation may be used for preventing current from accidentally reaching ground through a human or animal subject, e.g. the body of a patient. Further, in medical technology and particularly in stimulation techniques, several types of galvanic isolation may be considered. The examples of the galvanic isolation may include, but may not be limited to, the following:

- Disconnecting a circuit to be applied to a patient from the main power supply (e.g. 230 V) for the safety of the patient;
- In case of using two or more current sources generating current signals, for preventing mutual influence on current paths to be formed in two or more practically independent circuits.

Moreover, galvanic isolation for the medical device 10 may be realized as either true galvanic isolation or virtual galvanic isolation.

In various embodiments and examples as described herein, the "true galvanic isolation" may refer to electrically isolating more than one power sources using totally electrically separated stimulation circuits.

Further, in various embodiments and examples as described herein, the "virtual galvanic isolation" may refer to electrically isolating more than one power sources without using existing galvanic isolation techniques but with appropriate control of each of the switches included in more than one circuits 12. For example, each of the switches may be set to the open or closed state so as to ensure only one power source can be active or operating. The exemplary control of the switches for realizing the "virtual galvanic isolation" will be described below in connection with current signal patterns.

< Signal Patterns >

The signal patterns of the power applied to the circuit 12 of the medical device 10 will be described below.

First, the difference between the current signal ipattern and the actual current that may flow between the electrodes should be noted. The current signal $i_{pattern}$ may be the current signal that is generated by the current source connected to the power terminal PT of the circuit 12. The actual current between the electrodes, e.g. the actual stimulation current, may be the sampled signal of the current signal $i_{pattern}$. For example, referring to Table 1 and Fig. 3, assume that the switches S1, S2, S3 and S4 are set to operate the medical device 10 in the stimulation mode and in the inactive or short circuit mode alternately in a certain frequency while the current source is providing the current signal $i_{pattern}$ to the power terminal PT. In this exemplary operation, the current signal $i_{pattern}$ will be applied between the first and second electrode terminals E1, E2 only during the stimulation mode at the certain frequency. In other words, the actual current applied between the first and second electrode terminals E1, E2 will be the sampled current signal $i_{pattern}$ with a sampling rate of the certain frequency.

Depending on the sampling rate, direct current (DC), low frequency (LF), medium frequency (MF) or high frequency (HF) stimulation through applying current can be made on a human or animal subject. For example, in case of DC stimulation, the frequency of stimulation may be 0 Hz. Further, for example, the LF stimulation may refer to a frequency range between 1 Hz to 1000 Hz and the MF stimulation may refer to a frequency range between 1000 Hz to 100000 Hz. The HF stimulation, which may be used for e.g. thermotherapy, may involve a frequency higher than 100000 Hz, for example. It should be noted that the LF stimulation can involve both monophasic pulses and biphasic pulses as the current signal. For the MF stimulation, in practice, only biphasic pulses may be employed. The use of the monophasic or biphasic pulses from circuits with a virtual galvanic isolation may be the most cost effective implementation of the medical device 10.

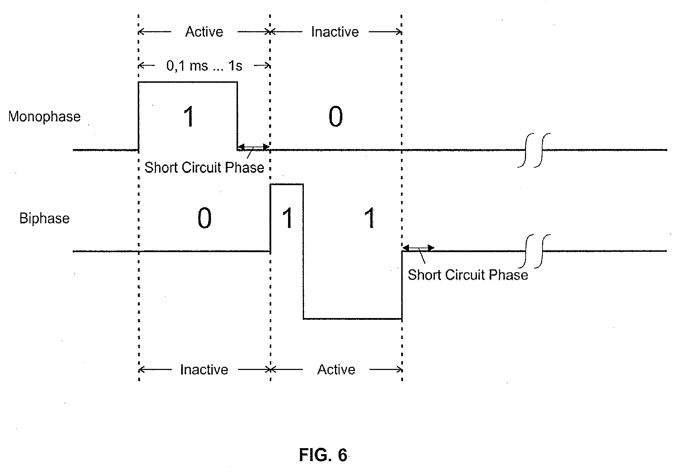

**Fig. 6** shows examples of a monophasic pulse signal and a biphasic pulse signal. The upper signal shown in Fig. 6 is an exemplary monophasic pulse signal that may be provided to the circuit 12 as the current signal. The lower signal shown in Fig. 6 is an exemplary biphasic pulse signal that may be provided to the circuit 12 as the current signal. The short circuit phases indicated in Fig. 6 may be implemented by setting the states of the switches of the circuit receiving the corresponding pulse signal as shown in the "short circuit" mode of Table 1.

In one example, one of the exemplary monophasic and biphasic pulse signals shown in Fig. 6 may be applied to the power terminal PT0 of the first circuit C0 shown in Fig. 5 and the other one of the exemplary monophasic and biphasic pulse signals shown in Fig. 6 may be applied to the power terminal PT1 of the second circuit C1 shown in Fig. 5. In this example, as can be seen from Fig. 6, while the channel for the monophasic pulse signal is active, the channel for the biphasic pulse signal may be made inactive and vice versa. The controller 20 may control the switches of the first and second circuits C0, C1 of the medical device 10 so that an electrode terminal of the circuit corresponding to the active pulse signal is electrically connected to the current source and the electrode terminals of the circuit corresponding to the inactive pulse signal is electrically disconnected from the current source. The short circuit phases shown in Fig. 6 may guarantee a secure transition between different switch settings of the first and second circuits C0, C1 and/or enable the charge flowed during the flow of the stimulation current to be substantially drawn out of the circuit that has had the stimulation current flowed through. This may be useful in, for example, skin regeneration. In case the stimulation current is free of direct current, which can be caused either by the offset current of the current source or by the averaged DC of the monophasic current flow, the short circuit phases for removing the charge may not need to be introduced.

Instead of the monophasic and/or biphasic pulse signals, current signals with other patterns or forms may be employed for applying to the power terminal PT of the circuit 12.

**Fig. 7** shows exemplary signal patterns of the current signals that may be applied to the circuit(s) 12 of the medical device 10 in case of LF stimulation. Fig. 7 shows exemplary signal patterns of two current sources, one generating a current signal $i_{pattern1}$ for channel 1 and the other generating a current signal $i_{pattern2}$ for channel 2. Each of the channels 1 and 2 may be electrically connected to the power terminal PT of different one of the circuits 12 comprised in the medical device 10. The curves with dashed and dotted lines of the current signals $i_{pattern1}$ and $i_{pattern2}$ shown in Fig. 7 represent the envelope curves generated by respective current sources and switch-settings.

Parts of the envelope curves during time periods indicated by the hatched areas in Fig. 7 represent the actual signal forms used for stimulation. During the time periods of stimulation indicated by hatched areas in Fig. 7, the controller 20 may control the medical device 10 to set the switches of the circuits 12 so that an electrode terminal of the corresponding circuit is electrically connected to the power terminal PT of that circuit. As shown in Fig. 7, the controller 20 may control the medical device 10 so that only one circuit 12 will be in the time period of stimulation at a time. In other words, when one power source channel is active for stimulation, the other power source channel is made inactive by setting the circuit connected to the other channel in the inactive mode shown in Table 1. In Fig. 7, $\Delta T$ indicates a cycle in which the stimulation may be performed. Thus, $1/\Delta T$ may be understood as indicating the sampling frequency of the current signal.

The short circuit phases are also introduced for the exemplary current signals shown in Fig. 7, similarly to the example of Fig. 6. As stated above with reference to Fig. 6, the short circuit phases may be implemented by setting the switches of the relevant circuit as shown in the "short circuit" mode in Table 1. In the example of Fig. 7, a short circuit phase may be set from the end of a time period of stimulation (indicated by a hatched area) for one channel until the start of a time period of stimulation for the other channel. In other words, a time period of stimulation for one channel may start when the short circuit phase for the other channel is over. During the short circuit phase, the remaining charge within the circuit may be drawn out to the ground, in other words, removed from the circuit as described above. Thus, after a short circuit phase, the circuit connected to that channel may change in the inactive mode and become high impedance against the other channel. Accordingly, the introduction of the inactive mode as shown in Fig. 7 can realize virtual galvanic isolation between the two circuits connected to the two channels, eliminating the need of true galvanic isolation.

In case the medical device 10 includes only one circuit 12 (see e.g. Fig. 3), one of the two current signals $i_{pattern1}$ and $i_{pattern2}$ shown in Fig. 7 may be applied to the power terminal PT of the circuit 12. Further, in case the medical device 10 includes more than one circuits 12 but uses a single current source (see e.g. Fig. 4), one of the two current signals $i_{pattern1}$ and $i_{pattern2}$ shown in Fig. 7 may be provided to the circuits 12.

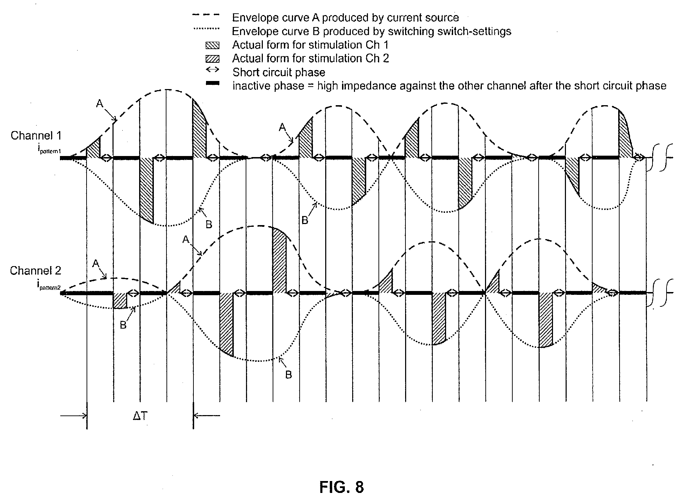

Increasing the sampling frequency by reducing the cycle $\Delta T$ shown in Fig. 7, for example, over about 1.0 kHz, MF stimulation current signals as shown in **Fig. 8** may be considered. Fig. 8 shows exemplary signal patterns of the current signals that may be applied to the circuit(s) 12 of the medical device 10 in case of MF stimulation. Similarly to Fig. 7, Fig. 8 shows exemplary signal patterns of two current sources, one generating a current signal $i_{pattern1}$ for channel 1 and the other generating a current signal $i_{pattern2}$ for channel 2. The curves with dashed and dotted lines of the current signals pattern1 and $i_{pattern2}$ shown in Fig. 8 represent the envelope curves generated by respective current sources. For each of the current signals $i_{pattern1}$ and $i_{pattern2}$, curves with a dashed line A and a dotted line B are shown in Fig. 8. The curve with the dotted line B represents a current signal having an opposite direction from the current signal represented by the curve with the dashed line A. The change of directions of the current signal may be realized by changing the settings of the switches of the circuit 12. For example, referring to Table 1, by changing the switch settings from that of the stimulation mode (E1 $\rightarrow$ E2) to that of the stimulation mode (E2 $\rightarrow$ E1) or vice versa, the direction of the stimulation current would become opposite. Parts of the envelope curves during time periods indicated by the hatched areas in Fig. 8 represent the actual signal forms used for stimulation, similarly to the examples of Fig. 7.

The functions and effects of the short circuit phases shown in Fig. 8 may be identical to that of the short circuit phases shown in Fig. 7 as stated above.

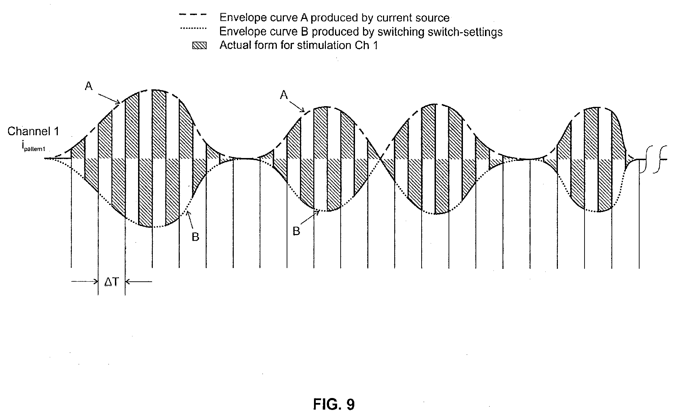

In case only one current source is used for the medical device 10, for example, when the medical device 10 includes a single circuit 12 (see e.g. Fig. 3) or more than one circuits 12 receiving power from a single current source (see e.g. Fig. 4), the short circuit phases may be omitted as shown in **Fig. 9.** Fig. 9 shows yet another exemplary signal pattern of the current signal that may be applied to the circuit 12 of the medical device 10 in case of MF stimulation. As in Fig. 8, the curves with dashed lines in Fig. 9 represent the envelope curves generated by the current source and the curve with dotted line B represents a current signal having an opposite direction from the current signal represented by the curve with the dashed line A. The change of directions of the current signal may be realized by changing the settings of the switches of the circuit 12 as stated above referring to Fig. 8 and Table 1. Parts of the envelope curves during time periods indicated by the hatched areas in Fig. 9 represent the actual signal forms used for stimulation. In Fig. 9, the signal pattern has no short circuit phase.

< Current Paths >

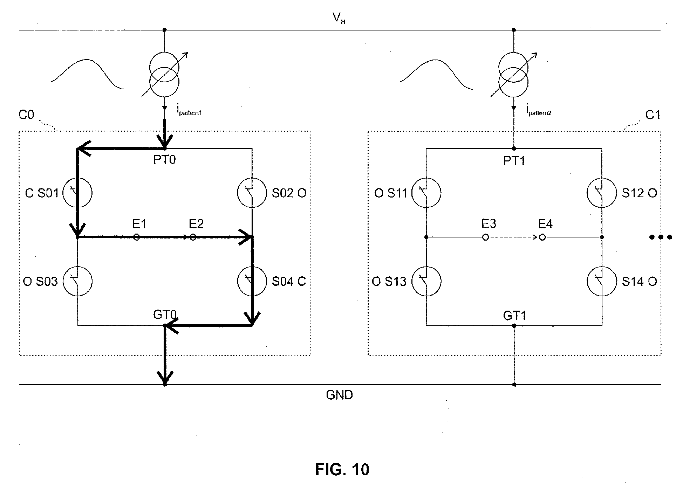

**Figs. 10 to 12** show exemplary paths of current during the stimulation mode of the medical device 10 having the circuits 12 as shown in Fig. 5. In Figs. 10 to 12, the character "o" shown next to a switch indicates that the switch is set to the open state and the character "c" shown next to a switch indicates that the switch is set to the closed state. As mentioned above with reference to Fig. 3, no current or only a negligible amount of current can flow through a switch in the open state while current can flow through a switch in the closed state.

**Fig. 10** shows an exemplary path of current when stimulation between a pair of electrodes connected to the electrode terminals E1 and E2 in the first circuit C0 is performed. In this example, the first switch S01 and the fourth switch S04 of the first circuit C0 are set to the closed state and all other switches in the first and second circuits C0, C1 are set to the open state. The current may flow from the current source connected to the power terminal PT0 of the first circuit C0 to the electrode terminal E1 via the closed switch S01, and then to the electrode terminal E2 via the pair of electrodes and a human or animal subject which the pair of electrodes are applied to. The current flowed into the electrode terminal E2 may further flow to the ground via the closed switch S04 and the ground terminal GT0 of the first circuit C0. It should be noted that, in the example shown in Fig. 10, the electrode terminals E3 and E4 of the second circuit C1 are electrically disconnected from the power source and the ground terminal GT1 by setting the switches S11, S12, S13 and S14 to the open state.

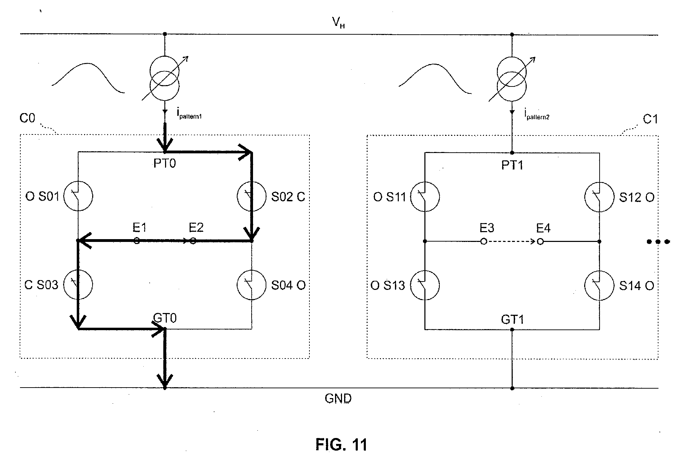

**Fig. 11** shows another exemplary path of current when stimulation between a pair of electrodes connected to the electrode terminals E1 and E2 in the first circuit C0 is performed. In this example, however, the current may flow in a direction opposite from that in the example shown in Fig. 10. In the example shown in Fig. 11, the second switch S02 and the third switch S03 of the first circuit C0 are set to the closed state and all other switches in the first and second circuits C0, C1 are set to the open state. The current may flow from the current source connected to the power terminal PT0 of the first circuit C0 to the electrode terminal E2 via the closed switch S02, and then to the electrode terminal E1 via the pair of electrodes and a human or animal subject which the pair of electrodes are applied to. The current flowed into the electrode terminal E1 may further flow to the ground via the closed switch S03 and the ground terminal GT0 of the first circuit C0. It should be noted that, also in the example shown in Fig. 11, the electrode terminals E3 and E4 of the second circuit C1 are electrically disconnected from the power source and the ground terminal GT1 by setting the switches S11, S12, S13 and S14 to the open state.

Even in case only one current source is made active and that current source provides only positive currents, the direction of the stimulation current between a pair of electrodes may be changed by changing the switch settings between the one shown in Fig. 10 and the one shown in Fig. 11, for example.

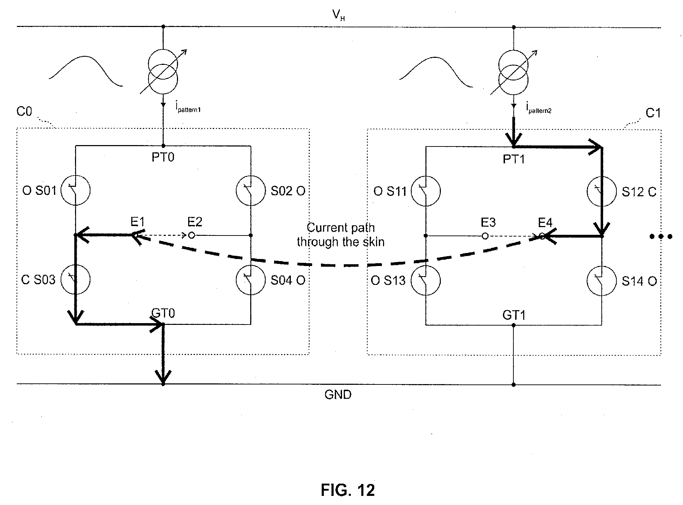

**Fig. 12** shows an exemplary path of current when stimulation between a pair of electrodes connected to the electrode terminal E1 in the first circuit C0 and the electrode terminal E4 in the second circuit C1 is performed. In this example, the second switch S12 of the second circuit C1 arranged between the power terminal PT1 and the electrode terminal E4 of the second circuit C1 is set to the open state. Further, the third switch S03 of the first circuit C0 arranged between the electrode terminal E1 and the ground terminal GT0 of the first circuit C0 is set to the open state. All other switches in the first and second circuits C0, C1 are set to the open state. The current may flow from the current source connected to the power terminal PT1 of the second circuit C1 to the electrode terminal E4 via the closed switch S12, and then to the electrode terminal E1 of the first circuit C0 via the pair of electrodes and a human or animal subject which the pair of electrodes are applied to. The current flowed into the electrode terminal E1 may further flow to the ground via the closed switch S03 and the ground terminal GT0 of the first circuit C0. It should be noted that, in the example shown in Fig. 12, the electrode terminals E1 and E2 of the first circuit C0 are electrically disconnected from the power source by setting the first and second switches S01, S02 to the open state.

Figs. 10 to 12 merely show some of the examples of possible current paths during the stimulation mode of the medical device 10 having more than one circuits 12. Stimulation can be made by a pair of electrodes connected to any arbitrary pair of electrode terminals selected from the electrode terminals in the first and second circuits C0, C1 (e.g., any pair selected from electrode terminals E1 to E4). The controller 20 may control the medical device 10 to set each switch in the first and second circuits C0, C1 to the closed state or the open state so as to:

- electrically connect one of the selected pair of electrode terminals to the power terminal PT of the circuit comprising said one of the selected pair of electrode terminals;
- electrically connect another one of the selected pair of electrode terminals to the ground terminal GT of the circuit comprising the other one of the selected pair of electrode terminals; and
- electrically disconnect the electrode terminals other than the selected pair of electrode terminals from the ground terminal GT and from the power terminal PT.

According to the control of switch settings as stated above, the current may flow from the power source to one of the selected pair of electrode terminals that is electrically connected to the power terminal PT (thus to the power source), and then, via the pair of electrodes applied to a human or animal subject, to the other one of the selected pair of electrode terminals that is electrically connected to the ground terminal GT (thus to the ground). The current may further flow to the ground from the other one of the selected pair of electrode terminals.

Although Figs. 10 to 12 show two circuits 12 in the medical device 10, the control of the switch settings as stated above may be analogously applicable to the medical device 10 including three or more circuits 12.

Further, although the exemplary current paths and the control of switch settings are described above in connection with the circuits 12 shown in Fig. 5, the same may apply to the circuits 12 shown in Fig. 4 where all the circuits 12 receive power from a single power source.

As stated above, the medical device 10 including more than one circuits 12 may enable stimulation using any selected pair of electrode terminals. The direction of the stimulation current between the selected pair of electrode terminals may also be selected by appropriately selecting which one of the selected pair of electrode terminals to be connected to the power terminal or the ground terminal. Accordingly, with the medical device 10 including more than one circuits 12, for example, once four or more electrodes are applied to a human or animal subject and each of the electrodes is connected to one of the electrode terminals of the circuits 12 included in the medical device 10, desired stimulation on selected nerve may be performed without changing the placements of the electrodes.

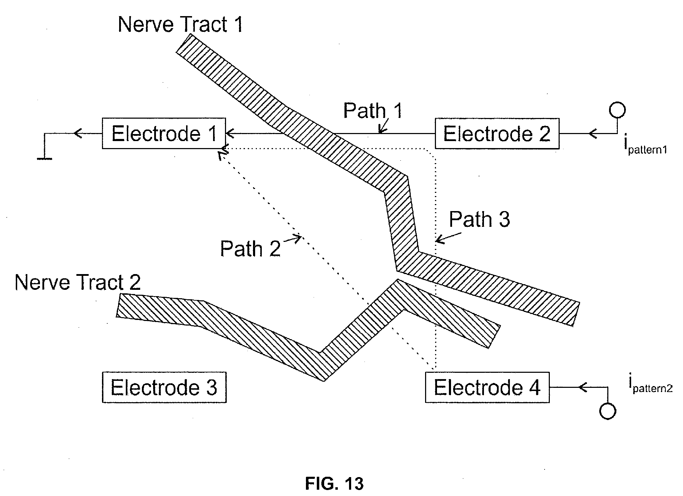

For instance, **Fig. 13** shows an example of current paths when stimulation is performed using the medical device 10 with the first and second circuits C0, C1 as shown in Fig. 4 or 5. The electrodes 1 to 4 shown in Fig. 13 indicate the arrangement of the four electrodes applied to a human or animal subject. The electrodes 1 to 4 shown in Fig. 13 may be connected to the electrode terminals E1, E2, E3 and E4 as shown in Fig. 4 or 5, respectively. The nerve tracts 1 and 2 in Fig. 13 indicate exemplary nerve tracts of the human or animal subject.

For example, suppose it is desired to stimulate only the nerve tract 1 and to make the stimulation current to flow in a direction from the electrode 2 to the electrode 1 shown in Fig. 13. Then the electrode terminals E1 and E2 respectively connected to the electrodes 1 and 2 may be selected and used for stimulation. In this case, the controller 20 may control the medical device 10 to set the switches of the first and second circuits C0, C1 to the states as shown in Fig. 11 so that the stimulation current may flow from the current source generating the current signal $i_{pattern1}$ to the electrode 2 and then to electrode 1, stimulating the nerve tract 1 (see path 1 of Fig. 13).

In another example, only the nerve tract 2 may be desired to be stimulated with stimulation current flowing in a direction from the electrode 4 to the electrode 1. In this example, the electrode terminals E1 and E4 respectively connected to the electrodes 1 and 4 may be selected and the switches of the first and second circuits C0, C1 of the medical device 10 may be set to the states as shown in Fig. 12. Then the stimulation current may flow from the current source generating the current signal $i_{pattern2}$ to the electrode 4 and then to electrode 1, stimulating the nerve tract 2 (see path 2 of Fig. 13).

In yet another example, suppose it is desired to stimulate both of the nerve tracts 1 and 2 with stimulation current flowing in a direction from the electrode 4 to the electrode 2 and subsequently to stimulate the nerve tract 1. In this example, first the electrode terminals E2 and E4 respectively connected to the electrodes 2 and 4 may be selected for stimulation. In order for making the stimulation current to flow in a direction from the electrode 4 to the electrode 2, the controller 20 may control the medical device 10 to: set the second switch S12 of the second circuit C1 to the closed state for electrically connecting the electrode terminal E4 to the power terminal PT1 of the second circuit C1; set the fourth switch S04 of the first circuit C0 to the closed state for electrically connecting the electrode terminal E2 to the ground terminal GT0 of the first circuit C0; and set all other switches- to the open state for electrically disconnecting the electrode terminals E1 and E3 from the power terminal and the ground terminal (see Fig. 4 or 5). With such settings of the switches, the stimulation current may flow from the current source generating the current signal $i_{pattern2}$ to the electrode 4 and then to electrode 2, stimulating the nerve tracts 1 and 2 (see path 3 of Fig. 13). Subsequently, the controller 20 may change the switch settings to that shown in Fig. 11 in order for the stimulation current to flow from the electrode 2 to the electrode 1 via the nerve tract 1 (see path 3 of Fig. 13).

< Advantages >

The medical device 10 including circuit(s) 12 having one of the exemplary configurations of Figs. 3 to 5 may enable electrical stimulation of a DUT (device under test) with a desired form of current signal flowing in a desired direction. As stated above, the direction of the stimulation current may be set only by controlling the states of the switches in the circuit(s) 12. In particular, when both electrodes for stimulation are connected to the electrode terminals included in the same circuit 12 and this circuit 12 is connected to a desired current source, simply changing the open/closed states of the pair of switches (S1, S4) and (S2, S3) may make the desired current signal to flow in an opposite direction from the direction before the change of the switch settings. In other words, the medical device 10 including circuit(s) 12 having one of the exemplary configurations of Figs. 3 to 5 may easily realize electrical stimulation, regardless of the type of current direction (e.g. direct, monophasic or biphasic), the frequency of stimulation (e.g. DC, LF, MF and HF) and the form of the stimulation signal (e.g. arbitrary superposed with a modulation degree). This can contribute to saving costs for implementing the medical device 10.

Further, as described above with reference to Fig. 13, the medical device 10 with more than one circuits 12 may enable stimulation in different current paths without changing the arrangement of the electrodes. This may be useful when stimulation of selected nerves of a human or animal subject is required.

Moreover, with the medical device 10 including circuit(s) 12 having one of the exemplary configurations of Figs. 3 to 5, both LF stimulation and MF stimulation may be performed using the same circuit(s) 12, which can lead to cost effective implementation of the medical device 10. The introduction of the short circuit phase (see e.g. Figs. 6 and 7) allows the stimulated section to recover. Controlling the switches of the circuits 12 to have only one power source channel to be active may achieve virtual galvanic isolation, which can also lead to saving costs since no true galvanic isolation may be necessary. Under the virtual galvanic isolation it may be ensured that only one power source can affect the circuits 12 of the medical device 10 at a time.

**Embodiment 2: Circuit with a Ground Connecting Switch**

< Basic Circuit Configuration and Switch Control >

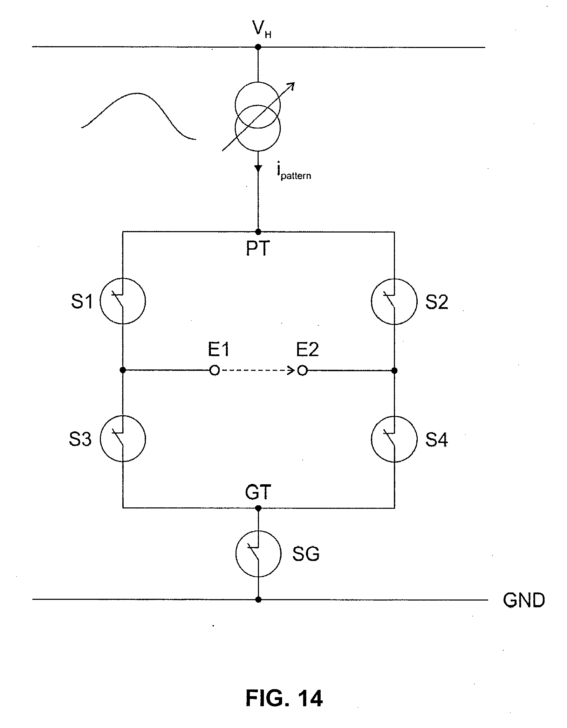

**Fig. 14** shows an example of the circuit 12 that may be included in the medical device 10 according to Embodiment 2. The exemplary circuit shown in Fig. 14 is identical to that shown in Fig. 3 except that the exemplary circuit shown in Fig. 14 further includes a ground connecting switch SG that is electrically connected to the ground terminal GT. The ground connecting switch SG may be configured to electrically connect or disconnect the ground terminal GT to the ground. Further, the ground connecting switch SG may be configured to set its state according to a control signal received from the controller 20. The control signal may indicate whether the ground connecting switch SG should be set to the open state or the closed state.

When the ground connecting switch SG as well as the first and second switches S1, S2 are set to the open state and the third and fourth switches S3, S4 are set to the closed state, the first and second electrode terminals E1, E2 will be connected to each other (in other words, short circuited) but will not be connected to the ground. Consequently, with such a setting of the switches, the first and second electrode terminals will be forced to have the same potential and no current can flow through a part of the circuit 12 to GND including the first and second electrode terminals E1, E2 as well as the third and fourth switches S3, S4.

Also in case the medical device 10 includes the circuit 12 having an exemplary configuration shown in Fig. 14, the controller 20 may control the medical device 10 to operate in one of the exemplary modes shown in Table 1: stimulation mode (E1 → E2), stimulation mode (E2 → E1), inactive mode and short circuit mode. The settings of the switches S1, S2, S3 and S4 may be identical to those shown in Table 1. With the example of the circuit 12 shown in Fig. 14, the ground connecting switch SG may be set to the closed state in the stimulation modes (E1 → E2; E2 → E1) and in the inactive mode. Further, the ground connecting switch SG shown in Fig. 14 may be set to the open state in the short circuit mode. By setting the ground connecting switch SG shown in Fig. 14 to the open state in the short circuit mode, the circuit 12 may have a high impedance to the ground GND. In the short circuit mode with an open ground connecting switch SG, a current may flow through the short circuited part of the circuit 12 but not toward the ground through the ground terminal GT.

< Possible Extensions of the Circuit >

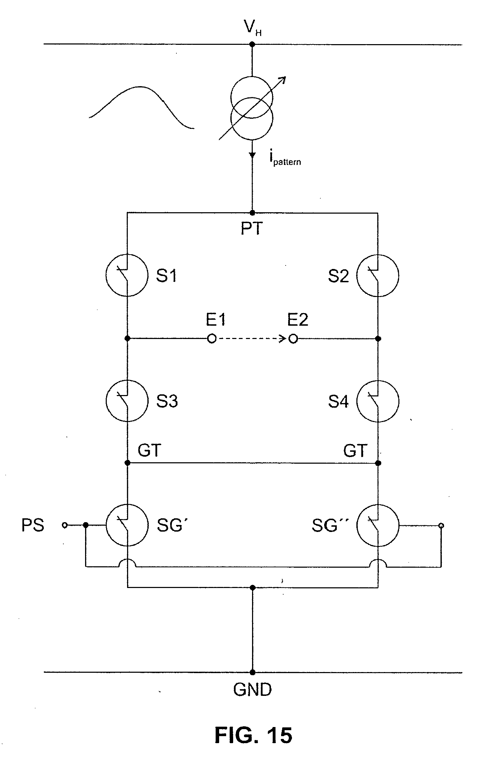

The ground connecting switch SG may be implemented by two switches electrically connected in parallel to the ground terminal GT as shown in **Fig. 15.** Fig. 15 shows another exemplary circuit with a pair of ground connecting switches SG', SG". The pair of ground connecting switches SG', SG" may be implemented in one or more ICs (integrated circuits) and/or electronic component(s). The pair of ground connecting switches SG', SG" may be configured to receive a control signal PS from the controller 20. In yet another example not shown in Fig. 15, the ground connecting switch SG may be implemented by three or more switches electrically connected to the ground terminal GT. The three or more switches functioning as the ground connecting switch SG may also be implemented in one or more IC(s) and/or electronic component(s).

Also in this Embodiment 2, the medical device 10 may include more than one circuits 12 having the exemplary configuration shown in Fig. 14 or 15.

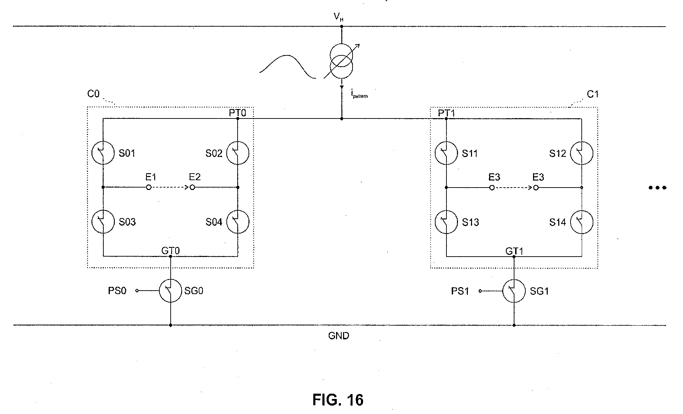

**Fig. 16** shows a part of exemplary configuration of the medical device 10 that includes more than one circuits 12 according to Embodiment 2. In Fig. 16, the medical device 10 may include a first circuit C0 and at least one second circuit C1 connected in parallel. Each of the first and second circuits C0, C1 shown in Fig. 16 has the exemplary circuit configuration as shown in Fig. 14. However, in yet another example, each of the first and second circuits C0, C1 may have the exemplary circuit configuration as shown in Fig. 15. The first and second circuits C0, C1 may be configured to receive input power from the same power source. For instance, in Fig. 16, the first and second circuits C0, C1 are connected to a single current source.

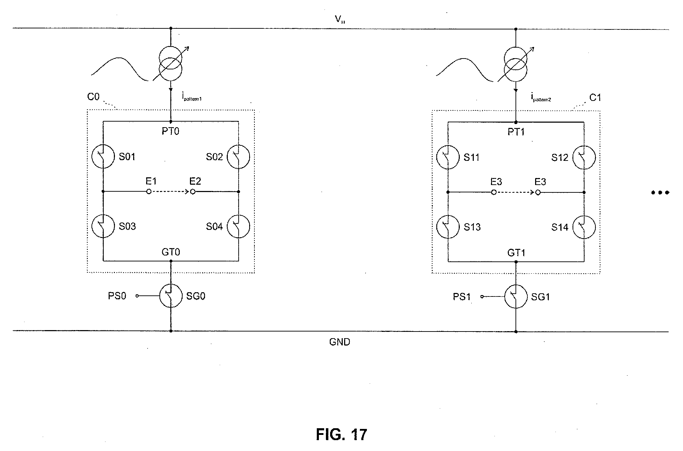

**Fig. 17** shows a part of another exemplary configuration of the medical device 10 that includes more than one circuits 12 according to Embodiment 2. In the example of Fig. 17, the medical device 10 has a configuration identical to that shown in Fig. 16 except that the first circuit C0 and the at least one second circuit C1 are configured to receive input power from different power sources. For example in Fig. 17, the power terminals PT0, PT1 of the first circuit C0 and of the at least one second circuit C1 are connected to different current sources that may be independent of each other.

When using two or more power sources for the medical device 10 as shown in Fig. 17, galvanic isolation must be considered, as explained above with reference to Fig. 5 of Embodiment 1. Virtual galvanic isolation of the circuits 12 receiving power from different power sources may be realized by controlling the states of the switches of the circuits 12 so that only one of the electrode terminals is electrically connected to the power terminal PT of the relevant circuit, only another one of the electrode terminals is electrically connected to the ground terminal GT of the relevant circuit and all other electrode terminals is electrically disconnected from the power terminal PT and the ground terminal GT.

< Signal Patterns >

Referring to Figs. 14 to 17, in the present Embodiment 2, the medical device 10 includes one or more circuit 12 having the ground connecting switch SG in addition to the first to fourth switches S1 to S4 that may be present also in the exemplary circuits 12 of Embodiment 1. As mentioned above with reference to Fig. 14, the ground connecting switch SG may be set to the open state during the short circuit mode of the medical device 10, which may prevent current to flow through a part of the circuit 12 to the ground GND.

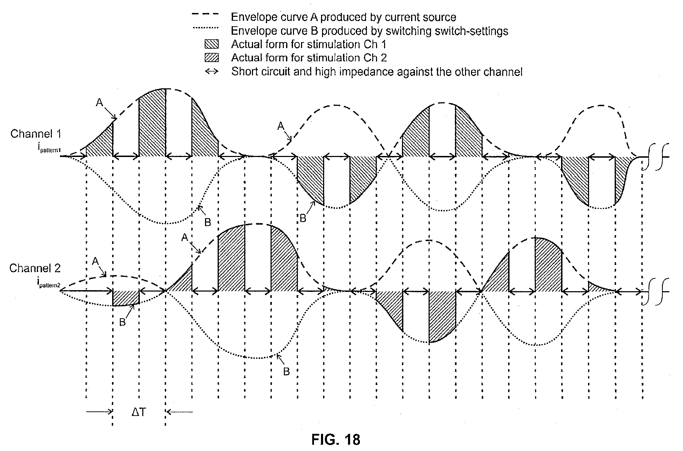

Accordingly, when employing more than one circuits 12 receiving power from different power sources, a time period of stimulation for one power source channel may start without waiting for the short circuit phase for another power source channel is over, in contrast to the exemplary signal patterns as described above with reference to Fig. 7 for the circuits 12 of Embodiment 1 without the ground connecting switch SG (see e.g. Figs. 3 to 5). Consequently, exemplary signal patterns of the current signals that may be applied to the circuit(s) 12 with the ground connecting switch SG may appear as shown in **Fig. 18.** Fig. 18 shows exemplary signal patterns in case of LF stimulation. Like in Fig. 7, Fig. 18 shows exemplary signal patterns of two current sources for channels 1 and 2. Further like in Fig. 7, the curves with dashed and dotted lines of the current signals $i_{pattern1}$ and $i_{pattern2}$ shown in Fig. 18 represent the envelope curves generated by respective current sources and switch-settings and the hatched areas in Fig. 18 indicate the actual signal forms used for stimulation. As can be seen from Fig. 18, a time period of stimulation for one channel can start without waiting for the short circuit phase of the other channel is over. Compared to the signal patterns shown in Fig. 7, the cycle ΔT for sampling may be made shorter and stimulation may be performed longer with the signal patterns shown in Fig. 18.

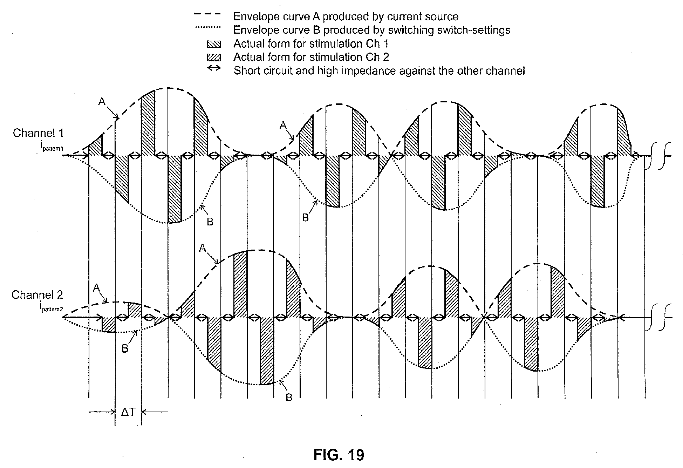

**Fig. 19** shows other exemplary signal patterns in case of MF stimulation using the circuit(s) 12 with the ground connecting switch SG. Also in Fig. 19, exemplary signal patterns of two current sources for channels 1 and 2 are shown. Like in Figs. 7, 8 and 18, the curves with dashed and dotted lines of the current signals $i_{pattern1}$ and $i_{pattern2}$ shown in Fig. 19 represent the envelope curves generated by respective current sources and switch-settings and the hatched areas shown in Fig. 19 indicate the actual signal forms used for stimulation. Further, like in Fig. 8, for each channel, the curve with the dotted line B represents a current signal having an opposite direction from the current signal represented by the curve with the dashed line A. The change of directions of the current signal may be realized by changing the settings of the switches of the circuit 12 in a way analogous to that as explained above in connection with Embodiment 1.

In case only one power source is employed for one or more circuits 12, one of the exemplary signal patterns of channels 1 and 2 shown in Figs. 18 and 19 may be used as the current signal to be applied to the power terminal(s) PT of the circuit(s) 12.

< Current Paths >

The exemplary current paths during the stimulation mode of the medical device 10 as described above with reference to Figs. 10 to 13 in connection with the Embodiment 1 may apply analogously to the medical device 10 of the Embodiment 2, wherein the circuit 12 includes the ground connecting switch SG.

< Advantages >

The medical device 10 including the circuits 12 with the exemplary configuration shown in Fig. 17 may be realized with a simple implementation, for example, using two quad-MOS switch IC and a dual-MOS switch IC as well as a resistance and a current source for two electrodes.

The medical device 10 including the circuit(s) 12 of this Embodiment 2 (see e.g., Figs. 14 to 17) may have the same advantages concerning the use of the four switches in the circuit 12, as described above with respect to Embodiment 1.

In addition, the additional ground connecting switch SG introduced in this Embodiment 2 may provide further protection for the human or animal subject. For example, while a power source channel is made inactive by controlling the switches of the circuit 12' connected to the relevant power source, the first and second switches S1, S2 as well as the ground connecting switch SG may be set to the open state and only the third and fourth switches S3, S4 may be set to the closed state. Such switch settings may short circuit the first and second electrode terminals E1, E2 and the discharge of the current that flowed earlier may occur. The discharge may stimulate a part of the skin of the human or animal subject to which the electrodes are applied, which may contribute to the recovery of the stimulated part of the skin. Moreover, in the same exemplary situation (S1, S2 and SG open; S3, S4 closed), no outer current can flow through the circuit 12 to the ground. Since the first and second electrode terminals E1, E2 are shorted, no current can flow through the skin path between the electrodes E1 and E2. It should be noted here, that the outer current can flow through the skin path and then through the shorted path between terminal E1 and terminal E2 of the inactivated circuit, then through the other skin path back to the ground of the activated circuit. (For example, referring to the Fig. 1, the electrode terminals E1 and E2 are shorted. The current source connected to the electrode terminal E3 can flow through the skin path between electrode terminal E3 and electrode terminal E1, then directly to electrode terminal E2, and through the skin path between electrode terminal E2 and electrode terminal E4, then to ground. The reason is that the current flows in the path of lower resistance.) Consequently, stimulation without true galvanic isolation but with virtual galvanic isolation of the current sources can be realized, if needed.

## Embodiment 3: Circuit with Six Switches and an Optional Ground Connecting Switch

The various examples of the medical device 10 having the exemplary circuits 12 as described above in connection with the Embodiments 1 and 2 do not have the function of impedance and/or potential measurement. The following will describe yet another embodiment of the medical device 10 that has the function of impedance and/or potential measurement in addition to the stimulation function as described above concerning the Embodiments 1 and 2.

< Basic Circuit Configurations and Switch Control >

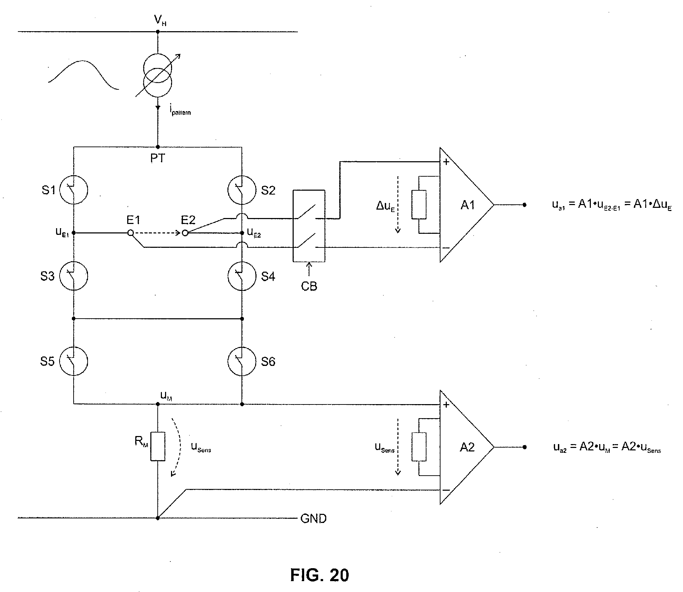

**Fig. 20** shows an example of the circuit 12 that may be included in the medical device 10 according to Embodiment 3. The exemplary circuit 12 shown in Fig. 20 may include six switches S1, S2, S3, S4, S5 and S6, a power terminal PT, a first electrode terminal E1, a second electrode terminal E2, a first differential amplifierA1, a second differential amplifier A2, a circuit breaker CB, a resistance $R_M$ and a ground terminal GT. The power terminal PT, the first to fourth switches S1 to S4 as well as the first and second electrode terminals E1, E2 in the circuit shown in Fig. 20 may be connected to each other in an analogous manner as the corresponding elements in the exemplary circuits as described above with reference to Figs. 3, 14 and 15.

In the exemplary circuit as shown in Fig. 20, each of the first and second electrode terminals E1, E2 is electrically connected to one of two input terminals of the first differential amplifier A1 via the circuit breaker CB. The circuit breaker CB may be configured to electrically disconnect the first differential amplifier A1 from the first and second electrode terminals E1, E2 in case a voltage applied between the first and second electrode terminals E1, E2 is higher than a specified (predetermined or predeterminable) threshold value. The specified threshold value may be determined in accordance with the characteristics of the differential amplifier A1. For example, the specified threshold value may be determined to have a value of the maximum voltage that the differential amplifier A1 can tolerate. The threshold value may be set based on the supply voltage of the operational amplifier employed as the differential amplifier A1. For example, for single supply, VCC may be 30 V and VEE may be 0V. An existing circuit breaker, for example, a mechanical circuit breaker, may be used as the circuit breaker CB of the circuit 12. In another example, the circuit breaker CB may be electronically realized using semiconductor technology, e.g. MOSFET or the like. An output of the first differential amplifier A1 may be an amplified value of the voltage between the first and second electrode terminals E1, E2.

Further according to Fig. 20, a first end of the third switch S3 may be electrically connected to the first electrode terminal E1 and a second end of the third switch S3 may be electrically connected to the ground terminal GT via the fifth switch S5 and the resistance $R_M$. A first end of the fourth switch S4 may be electrically connected to the second electrode terminal E2 and a second end of the fourth switch S4 may be electrically connected to the ground terminal

GT via the sixth switch S6 and the resistance $R_M$. The second end of the third switch S3 and the second end of the fourth switch S4 may be electrically connected to each other.

Further according to Fig. 20, a first end of the fifth switch S5 may be electrically connected to the second end of the third switch S3 and a second end of the fifth switch S5 may be electrically connected to a first end of the resistance $R_M$. Moreover, a first end of the sixth switch S6 may be electrically connected to the second end of the fourth switch S4 and a second end of the sixth switch S6 may be electrically connected to the first end of the resistance $R_M$. A second end of the resistance $R_M$ may be electrically connected to the ground terminal GT. Further, each of the first and second ends of the resistance $R_M$ may be electrically connected to one of two input terminals of the second differential amplifier A2. An output of the second differential amplifier A2 may be an amplified value of the voltage between the first and second ends of the resistance $R_M$.

With the exemplary circuit 12 shown in Fig. 20, the voltage drop $u_{Sens}$ across the resistance $R_M$ during the stimulation may be measured using the output of the second differential amplifier A2. Then the value of the current can be determined using the known value of the resistance $R_M$.

When all the switches S1 to S6 of the exemplary circuit 12 shown in Fig. 20 are set to the open state, signals between a pair of electrodes connected to the first and second electrode terminals E1, E2 may be measured with a part of the circuit 12 including the first and second electrode terminals E1, E2, the circuit breaker CB and the first differential amplifier A1. The circuit breaker CB must be set to the closed state to enable the signal measurement. For this signal measurement, the differential potential $\Delta u_E$ across the first differential amplifier A1 may be measured. The signal between the pair of electrodes, e.g. measured differential potential $\Delta u_E$, may be a biosignal (e.g. EMG, EEG) when the pair of electrodes are applied to a human or animal subject. The current source may automatically turn off itself when the first and second switches S1 and S2 are set to the open state.

As can be seen from Fig. 20, the circuit breaker CB and the first amplifier A1 as well as the resistance $R_M$ may function as a voltage divider when current flows from the power source to the ground through the circuit 12 via the first and second electrode terminals E1, E2 by setting either the first switch S1 or the second switch S2 to the closed state and appropriately setting the states of the rest of the switches.

In case of measuring an impedance (e.g. resistance) between a pair of electrodes connected to the first and second electrode terminals E1, E2, the current signal $i_{pattern}$ from the power source may flow between the first and second electrode terminals E1, E2, for example, through a skin section of a human or animal subject where the pair of electrodes are applied. The current signal $i_{pattern}$ may further flow through the known resistance $R_M$. In the example of measuring the impedance of the skin section of the human or animal subject, the impedance $R_{skin}$ of the skin section between the pair of electrodes connected to the first and second electrode terminals E1, E2 may be calculated as:

$$R_{skin} = \frac{\Delta u_E}{i_{skin}} \text{ (Equation 1)}$$

where the current through the skin section $i_{skin} = i_{pattern}$. The current through the skin section $i_{skin}$ may be determined as $U_{sens} / R_M$. Accordingly, the impedance $R_{skin}$ may be:

$$R_{skin} = \frac{\Delta u_E}{\frac{u_{Sens}}{R_M}} = \frac{\frac{u_{a1}}{A1}}{\frac{u_{a2}/A2}{R_M}} \text{ (Equation 2)}$$

where $u_{a1}$ and $u_{a2}$ may respectively indicate the output voltages of the first and second differential amplifiers A1, A2. Further in the above Equation 2, A1 and A2 may respectively indicate the amplification factors of the first and second differential amplifiers A1, A2. The amplification factors A1, A2 may be set to make signals adequate for the input dynamic range (e.g. [0 V ... 5 V]) of the following analog to digital converter (not shown).

The impedance measurement may be a resistance measurement, depending on the frequency of the source current signal $i_{pattern}$. The impedance of human skin may be measured from 0.1 Hz to 100 kHz.

During operation of the medical device 10 including the exemplary circuit 12 shown in Fig. 20, the circuit breaker CB may be set to the closed state, in other words, the circuit breaker CB may electrically connect the first differential amplifier A1 with the first and second electrode terminals E1, E2. When the voltage between the first and second electrode terminals E1, E2 becomes larger than a specified (predetermined or predeterminable) threshold value, however as mentioned above, the circuit breaker CB may be set to the open state and electrically disconnect the first differential amplifier A1 from the first and second electrode terminals E1, E2. This may protect the IC implementing the first differential amplifier from damages.

In order to deal with voltage values that may be required for operation in the stimulation mode, a differential amplifier with high voltage tolerance may be employed as the first differential amplifier A1. For example, a high common-mode voltage difference amplifier may be used as the first differential amplifier A1. The common mode input range of the amplifier may depend on the manufacturer of the amplifier. For example, INA117 from Texas Instrument may have $\pm 200$ V by supply voltage VS = $\pm 15$V and LT1990 from Linear technology may have $\pm$ 250 V by supply voltage VS = $\pm 15$V. Input protection can reach to $\pm$ 350 V. Employing a differential amplifier appropriate for high voltages as the first differential amplifier A1 may, however, decrease the input impedance of the first differential amplifier A1 as compared to the case when a differential amplifier with lower voltage tolerance is used as the first differential amplifier A1. When signal measurement is performed by setting all the switches S1 to S6 to the open state as mentioned above, the input impedance of the differential amplifier A1 may preferably be as high as possible, e.g. infinitely high. This is particularly true in case of biosignal measurement since the "signal source" of biosignals (e.g. a human or animal subject) cannot make a large value of current to flow between the electrode terminals E1, E2. From another point of view, if the input resistance of the differential amplifier becomes infinitely high, the full biosignal-voltage may be applied to the differential amplifier. The high impedance of the skin to electrode passage ahead of the amplifier can be neglected.

With the exemplary circuit 12 shown in Fig. 20, the voltage between the first and second electrode terminals E1, E2 may be monitored during operation in which current flows through the circuit 12 by setting either the first switch S1 or the second switch S2 to the closed state. However, the common-mode voltage at the end and the beginning of the first and second terminals E1, E2 must be kept within the voltage range of the first differential amplifier A1. Under this condition, impedance measurement as well as voltage and current monitoring may be performed during operation in which current flows through the circuit 12.

Table 2A shows exemplary possible modes of operation of the medical device 10 including the circuit 12 shown in Fig. 20.

Table 2A: Exemplary Operation Modes for the Circuit shown in Fig. 20

| Mode | Switch Settings |
|---|---|
| Inactive (High impedance between E1 & E2) | S1 to S4 open; S5 & S6 closed; and CB open. |
| High Impedance and Short Circuit | S1, S2, S5 & S6 open; S3 & S4 closed; and CB open. (This mode of operation may short circuit the first and second electrode terminals E1, E2 but at the same time make the circuit 12 in a state of high impedance since an outer current cannot flow through the circuit 12 toward the ground. The outer current may flow through the short-circuited part of the circuit 12 (including the electrode terminals E1, E2 and the switches S3 and S4) but not toward the ground through the ground terminal GT of the circuit 12. The outer current may flow away to the ground outside the circuit 12.) |
| Stimulation (between E1 & E2) | S1 & S4 open and S2 & S3 closed; or S1 & S4 closed and S2 & S3 open, while: S5 & S6 closed; and CB open. |
| Signal Measurement (e.g., EMG measurement between E1 & E2) | S1, S2, S3 & S4 open; S5 & S6 closed; and CB closed. A signal (e.g. EMG) may be measured with the first differential amplifier A1. |
| Impedance Measurement (between E1 & E2) | S1 & S4 open and S2 & S3 closed; or S1 & S4 closed and S2 & S3 open, while S5 & S6 closed; and CB closed. (see Equation 2) |

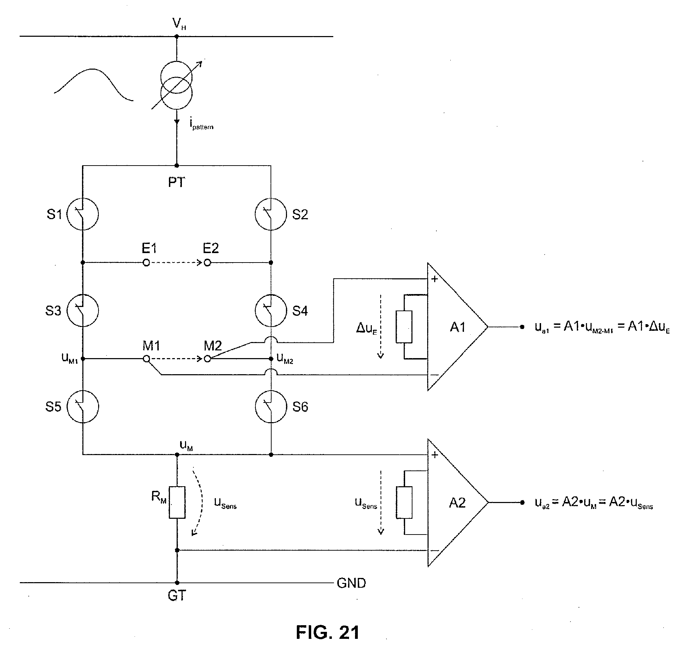

For improving the biosignal measurement function, the exemplary circuit 12 shown in Fig. 20 may be modified to obtain another exemplary circuit 12 shown in Fig. 21. The exemplary circuit 12 shown in Fig. 21 may include, like in the exemplary circuit 12 shown in Fig. 20, the power terminal PT, the first to sixth switches S1 to S6, the first and second electrode terminals E1, E2, the first and second differential amplifiers A1, A2, the resistance $R_M$ and the ground terminal GT. In the following, parts of the exemplary circuit 12 shown in Fig. 21 which are different from that shown in Fig. 20 will be explained.

The exemplary circuit 12 shown in Fig. 21 does not include the circuit breaker CB shown in Fig. 20. Instead, the exemplary circuit 12 shown in Fig. 21 may include a first measurement terminal M1 and a second measurement terminal M2. Each of the first and second measurement terminals M1, M2 may be electrically connected to one of two input terminals of the first differential amplifier A1. The first measurement terminal M1 may be arranged in between the third and fifth switches S3, S5. The first end of the third switch S3 may be connected to the first electrode terminal E1. The second end of the third switch S3 may be connected to the first measurement terminal M1 and to the first end of the fifth switch S5. The second measurement terminal M2 may be arranged in between the fourth and sixth switches S4, S6. The first end of the fourth switch S4 may be connected to the second electrode terminal E2. The second end of the fourth switch S4 may be connected to the second measurement terminal M2 and to the first end of the sixth switch S6.

In the exemplary circuit shown in Fig. 21, an output of the first differential amplifier A1 may be an amplified value of the voltage between the first and second measurement terminals M1, M2. Thus, when the fifth and sixth switches S5, S6 are set to the closed state so that both the first and second measurement terminals M1, M2 are electrically connected to the ground via the resistance $R_M$, the voltage between the first and second measurement terminals M1, M2 will be zero (or negligibly small), thereby preventing the first differential amplifier A1 from being subject to a voltage higher than it can tolerate.

In case of electrically stimulating a human or animal subject using the medical device 10 including the exemplary circuit 12 shown in Fig. 21, either the first switch S1 or the second switch S2 may be set to the closed state. When the first switch S1 is set to the closed state, the second and third switches S2, S3 must be set to the open state and the fourth switch S4 must be set to the closed state. When the second switch S2 is set to the closed state, the first and fourth switches S1, S4 must be set to the open state and the third switch S3 must be set to the closed state. In both cases, the fifth and sixth switches S5, S6 may be set to the closed state in order to protect the first differential amplifier A1 as stated above. During such stimulation operation, the current signal $i_{pattern}$ may flow through the circuit to the ground, via the first and second electrode terminals E1, E2 as well as the known resistance $R_M$. By monitoring the current that flows across the resistance $R_M$ using the output of the second differential amplifier A2, the amplitude of the current signal $i_{pattern}$ may be controlled in order to, for example, protect the human or animal subject and/or adjust the strength of the stimulation.

The medical device 10 including the exemplary circuit 12 shown in Fig. 21 may also be used for measuring bioelectric signals, for example, EMG, ECG, EEG, EOG, etc. During biosignal measurement, since the current signal $i_{pattern}$ should not flow into the first and second electrode terminals E1, E2, the first and second switches S1, S2 must be set to the open state. Further, the fifth and sixth switches S5, S6 may be set to the open state so as to prevent the bioelectric "signal source" to be influenced by the ground and to prevent the current to drain out through the resistance $R_M$. For optimization of the signals and suppression of distortion (for example, the common-mode distortion signal at about 50 Hz (or 60 Hz in the U.S.) could be problematic), it may be reasonable to differentially measure potentials at both the first and second electrode terminals E1, E2. For this measurement, the first and second measurement terminals M1, M2 connected to the first differential amplifier A1 may be provided. If approximately no current flows into the first differential amplifier A1 and resistances of the third and fourth switches S3, S4 that are set to the closed state are minor, the potentials $\Phi_{M1}$, $\Phi_{M2}$ at the first and second measurement terminals M1, M2 may be equal to the potentials ($\Phi_{E1}$, $\Phi_{E2}$ at the first and second electrode terminals E1, E2, respectively ($\Phi_{M1} = \Phi_{E1}$, $\Phi_{M2} = \Phi_{E2}$). Thus, the voltage $\Delta u_E$ between the electrode terminals E1, E2 may be determined from the potential difference. The first differential amplifier A1 may amplify the signal and suppress distortion.

A great advantage of the exemplary circuit 12 shown in Fig. 21 may be that this circuit can also be used for impedance measurement. In case impedance measurement is performed using the medical device 10 including the exemplary circuit 12 shown in Fig. 21, current signals $i_{pattern}$ with smaller amplitudes than the current signals $i_{pattern}$ used for the stimulation may be applied to the power terminal PT of the circuit 12. Thus, when the medical device 10 operates in the impedance measurement mode, the first differential amplifier A1 may not need to be separately protected (cf. Fig. 20). In the impedance measurement mode, while the third and fourth switches S3, S4 may always be set to the closed state, a pair of switches S1, S6 may be set to the closed state and the remaining pair of switches S2, S5 may be set to the open state. Alternatively, the pair of switches S1, S6 may be set to the open state and the pair of switches S2, S5 may be set to the closed state, while setting the third and fourth switches S3, S4 to the closed state. In some examples, the open and closed states of the pair of switches S1, S6 and the pair of switches S2, S5 may be changed from one another, if desired. The states of the switches may be controlled by the controller 20.

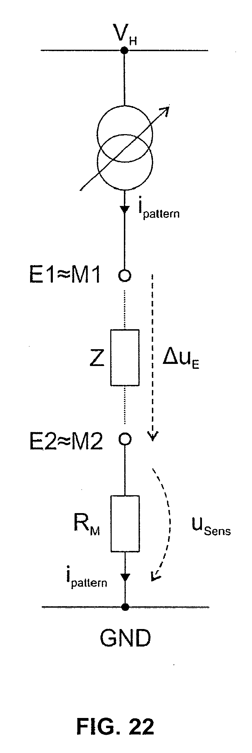

During the impedance measurement mode, the first differential amplifier A1 may deliver the voltage $\Delta u_E$ between a pair of electrodes connected to the first and second electrode terminals E1, E2. With the second differential amplifier A2, the voltage $u_{Sens}$ across the resistance $R_M$ may be measured and the current $i_{pattern}$ flowing through the resistance $R_M$ may be calculated from the measured voltage. Accordingly, the impedance Z between the pair of electrodes connected to the first and second electrode terminals E1, E2 may be determined. **Fig. 22** shows an example of a simplified circuit when the circuit shown in Fig. 21 is used for impedance measurement.

Table 2B shows exemplary possible modes of operation of the medical device 10 including the circuit 12 shown in Fig. 21.

Table 2B: Exemplary Operation Modes for the Circuit shown in Fig. 21

| Mode | Switch Settings |
|---|---|
| Inactive (high impedance between E1 & E2) | S1 to S4 open; S5 & S6 closed (for protecting the first differential amplifier A1 and to establish a ground reference for A1). |
| Short circuit (between E1 & E2) | S1 & S2 open; S3 to S6 closed (for protecting the first differential amplifier A1). |
| Stimulation (between E1 & E2) | S1 & S4 open and S2 & S3 closed; or S1 & S4 closed and S2 & S3 open, while: S5 & S6 closed (for protecting the first differential amplifier A1). |
| Signal Measurement (e.g., EMG measurement between E1 & E2) | S1, S2, S5 & S6 open; S3 & S4 closed. |
| Impedance Measurement (between E1 & E2) | S1 & S6 open and S2 & S5 closed; or S1 & S6 closed and S2 & S5 open, while S3 & S4 closed. (This mode of operation is possible without damaging the first differential amplifier A1 only if the amplitude of the current signal $i_{pattern}$ is made much smaller than that for stimulation operation.) |

< Possible Extensions of the Circuit >

Also in this Embodiment 3, the medical device 10 may include more than one circuits 12 having the exemplary configuration shown in Fig. 20 or 21.

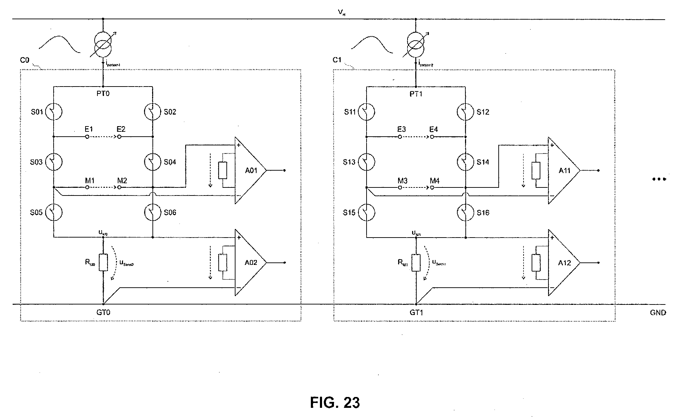

**Fig. 23** shows a part of yet another exemplary configuration of the medical device 10 that includes more than one circuits 12 according to the present Embodiment 3. In Fig. 23, the medical device 10 may include a first circuit C0 and at least one second circuit C1 connected in parallel. Each of the first and second circuits C0, C1 shown in Fig. 23 has the exemplary circuit configuration as shown in Fig. 21. However, in yet another example, each of the first and second circuits C0, C1 may have the exemplary circuit configuration as shown in Fig. 20. The first and second circuits C0, C1 may be configured to receive input power from different power sources. For example, in Fig. 23, the power terminal PT0 of the first circuit C0 is connected to a current source generating a current signal $i_{pattern1}$ and the power terminal PT1 of the second circuit C1 is connected to another current source generating a current signal $i_{pattern2}$.

Also with the exemplary configuration shown in Fig. 23, virtual galvanic isolation of the circuits 12 receiving power from different power sources may be realized by controlling the states of switches of the circuits 12 so that only one of the electrode terminals is electrically connected to the power terminal PT of the relevant circuit, only another one of the electrode terminals is electrically connected to the ground terminal GT of the relevant circuit and all other electrode terminals is electrically disconnected from the power terminal PT and the ground terminal GT.

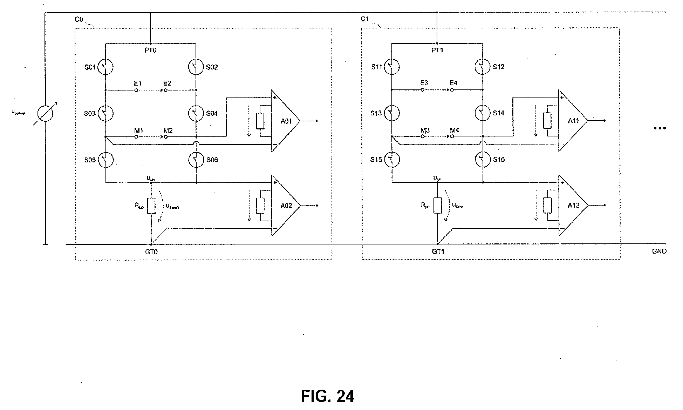

In other exemplary configurations, the first and second circuits C0, C1 may be configured to receive input power from a single power source. For example, referring to **Fig. 24,** the power terminals PT0, PT1 of the first and second circuits C0, C1 may be connected to a single voltage source that generates a voltage signal $u_{pattern}$. In yet another example, the power terminals PT0, PT1 of the first and second circuits C0, C1 may be connected to a single current source that generates a current signal $i_{pattern}$.

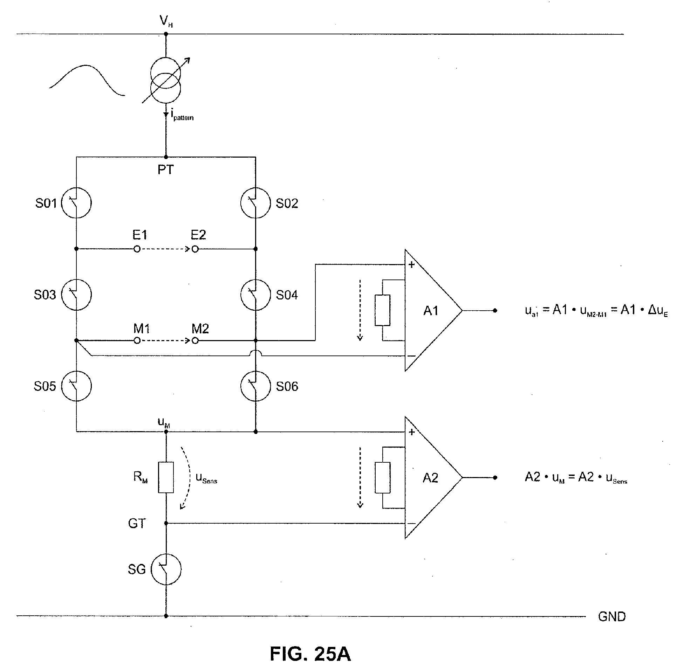

Further, in some exemplary configurations, each circuit 12 shown in Figs. 20, 21, 23 and 24 may additionally include a ground connecting switch SG as described above in Embodiment 2. By controlling the state of the ground connecting switch SG, the second end of the resistance $R_M$ may be electrically connected to or disconnected from the ground. For example, **Fig. 25A** shows an exemplary configuration where the circuit 12 shown in Fig. 21 additionally has a ground connecting switch SG. The exemplary circuit shown in Fig. 25A is identical to the circuit shown in Fig. 21 except that the ground connecting switch SG is provided between the resistance $R_M$ and the ground GND. In the example of Fig. 25A, the second end of the resistance $R_M$ may be considered as the ground terminal GT. Similarly to the Embodiment 2, the ground connecting switch SG shown in Fig. 25A may be configured to electrically connect or disconnect the ground terminal GT to the ground GND. In another example not shown in Fig. 25A, the ground connecting switch SG may be provided between the switches S5, S6 and the resistance $R_M$ for one end of the ground connecting switch SG to be electrically connected to the switches S5, S6 and the other end of the ground connecting switch SG to be electrically connected to the first end of the resistance $R_M$. In the exemplary configurations with a ground connecting switch SG in the Embodiment 3, the fifth and sixth switches S5, S6 of the circuit 12 can be electrically connected to the ground GND via the resistance $R_M$ and the ground connecting switch SG.

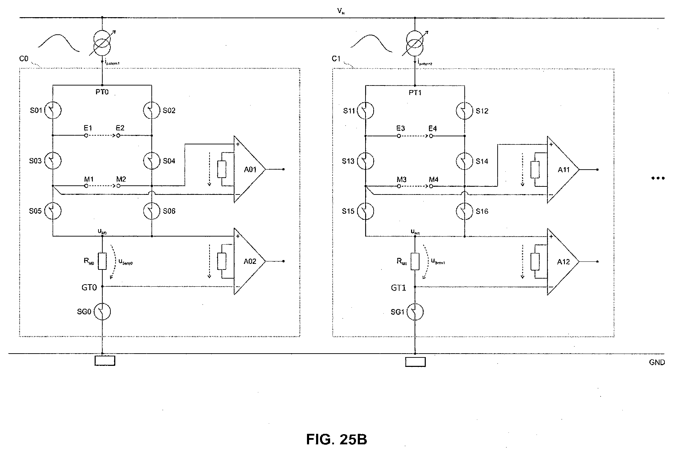

The inactive mode described in table 2B for the circuit C0 shown in Fig. 23 may realize a short circuit phase. This state, however, may not realize the high-ohmic state against the other circuit C1 anymore, due to the lack of the ground connecting switch SG. A small resistance $R_M$ connecting to the ground GND may be chosen, so that an outer current from C1 can flow through the circuit C0 to the ground GND. In this case, the virtual galvanic isolation between the circuits C0 and C1 - if suitable - may not be realized anymore. The circuit in the Fig. 25B shows a further extension of the circuit shown in Fig. 23. With the extended switch SG introduced in Fig. 14 the virtual galvanic isolation during the short circuit phase can be realized again.

When a ground connecting switch SG is provided so that the fifth and sixth switches S5, S6 of the circuit 12 (shown in e.g. Fig. 20, 21, 23 or 24) can be electrically connected to the ground GND via the resistance $R_M$ and the ground connecting switch SG, the resistance $R_M$ may have one of the following two states:

$$R_M = f(SG) = \begin{cases} R_M & if\ SG = 1 = closed\ state \\ \infty & if\ SG = 0 = open\ state \end{cases}$$

In case the medical device 10 includes the circuit having an exemplary configuration shown in Fig. 25A, the controller 20 may control the medical device 10 to operate in one of the exemplary modes shown in Table 2B: inactive mode, short circuit mode, stimulation mode, signal measurement mode and impedance measurement mode. The settings of the switches S1, S2, S3, S4, S5 and S6 may be identical to those shown in Table 2B. With the example of the circuit 12 shown in Fig. 25A, the ground connecting switch SG may be set to the closed state in the inactive mode, the stimulation mode, and the impedance measurement mode. In the short circuit mode, the ground connecting switch SG shown in Fig. 25A may be set to the open state. By setting the ground connecting switch SG to the open state during the short circuit mode, the circuit 12 may be in a state of high impedance while the first and second electrode terminals E1, E2 are short circuited. In this situation, an outer current cannot flow through the circuit 12 toward the ground GND, since the ground connecting switch is set to the open state. The outer current may flow through the short-circuited part of the circuit 12 (including the electrode terminals E1, E2 and the switches S3, S4, S5, S6) but not toward the ground via the ground terminal of the circuit 12. The outer current may flow away to the ground outside the circuit 12. In the signal measurement mode, the ground connecting switch SG shown in Fig. 25A may be set to either the closed state or the open state, since the switches S5, S6 are set to the open state and the terminals E1, E2 are already electrically disconnected from the ground.

**Fig. 25B** shows a part of yet another exemplary configuration of the medical device 10 that includes more than one circuits 12 according to the present Embodiment 3. In Fig. 25B, the medical device 10 may include a first circuit C0 and at least one second circuit C1 connected in parallel. Each of the first and second circuits C0, C1 shown in Fig. 25B has the exemplary circuit configuration as shown in Fig. 25A.

< Signal Patterns >

The exemplary signal patterns shown in Figs. 6 to 9, 18 and 19 as described above with respect to Embodiments 1 and 2 may be employed in an analogous manner for the circuit(s) 12 according to the present Embodiment 3. For instance, for the medical device 10 including the circuit(s) 12 without the ground connecting switch SG according to the present Embodiment 3 (see e.g. Figs. 20, 21, 23 and 24), the exemplary signal patterns as described above with reference to Figs. 6 to 9 concerning Embodiment 1 may be applied in an analogous manner. Further, for example, for the medical device including the circuit(s) 12 with the ground connecting switch SG according to the present Embodiment 3 (see e.g. Fig. 25A), the exemplary signal patterns as described above with reference to Figs. 18 and 19 concerning Embodiment 2 may be applied in an analogous manner.

For more specific example, in case of MF stimulation using the circuit(s) 12 including the ground connecting switch SG according to the present Embodiment 3 (see e.g. Fig. 25A), the exemplary signal patterns shown in Fig. 19 as described above with respect to Embodiment 2 may be employed. As can be seen from Fig. 19, the controller 20 may control the medical device 10 to receive power from only one power source channel at a time. In other words, only one power source channel may be made active at a time. While one power source channel is active and connected to one of the electrode terminals of the first and second circuits C0, C1 included in the medical device 10, the other power source channel must be made inactive, e.g. electrically disconnected from any of the electrode terminals of the first and second circuits C0, C1. Such control can achieve virtual galvanic isolation during the short circuit phase between the first and second circuits C0, C1 and eliminate the need of having true galvanic isolation for the circuits receiving power from different power sources.

The signal patterns of the current signal applied to the first and second circuits may take other forms than those shown in Fig. 19. As long as the controller 20 controls the medical device 10 to receive power from only one power source channel at a time, virtual galvanic isolation can be realized regardless of the forms and phases of the current signals.

< Current Paths >

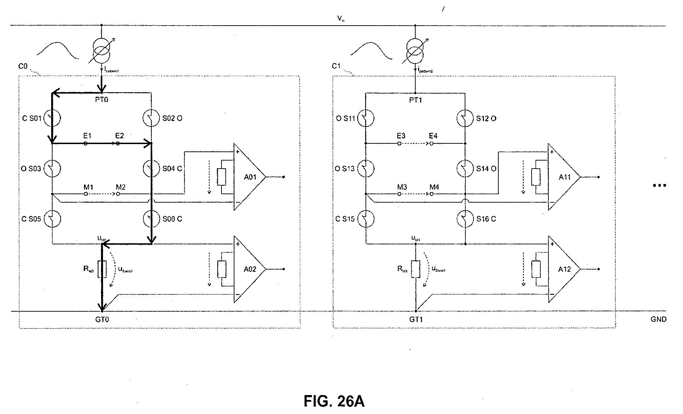

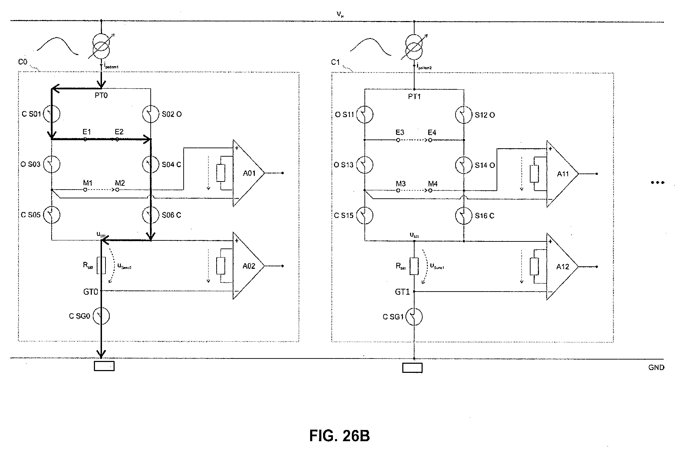

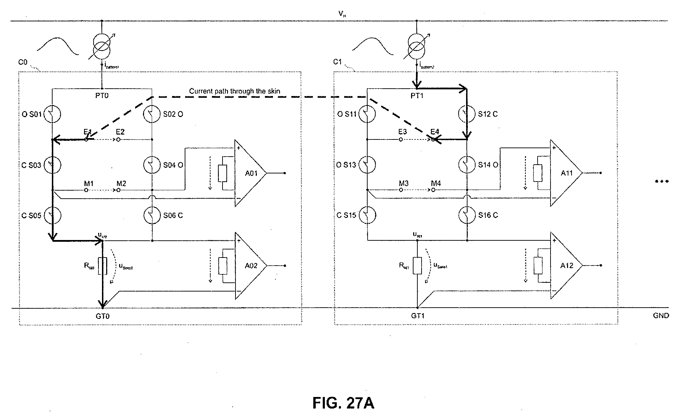

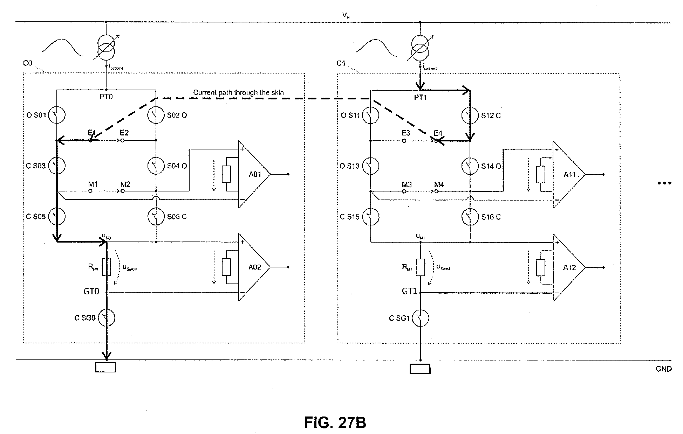

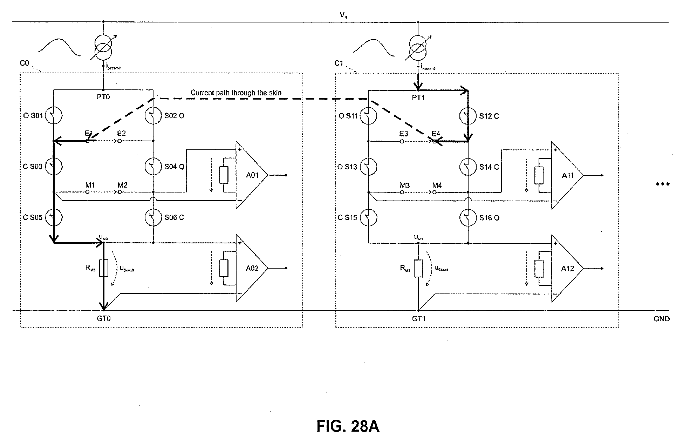

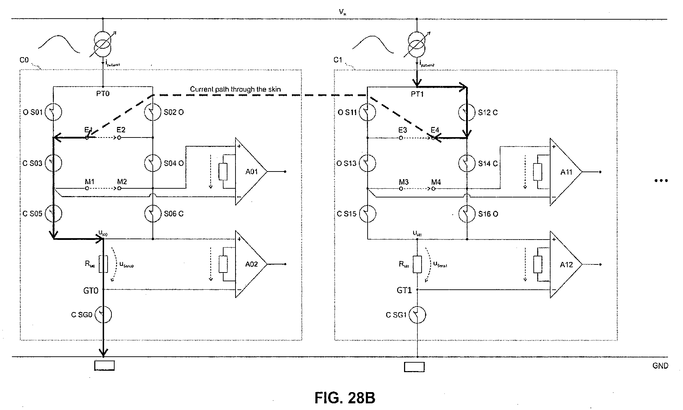

**Figs. 26A and 27A** show exemplary paths of current during the stimulation mode of the medical device 10 having the circuits 12 as shown in Fig. 23. Further, **Figs. 26B and 27B** show exemplary paths of current during the stimulation mode of the medical device 10 having the circuits 12 as shown in Fig. 25B. Further, **Figs. 28A and 28B** show exemplary paths of current during the impedance measurement mode of the medical device 10 having the circuits 12 as shown in Figs. 23 and 25B, respectively. In Figs. 26A to 28B, the character "o" shown next to a switch indicates that the switch is set to the open state and the character "c" shown next to a switch indicates that the switch is set to the closed state.

**Figs. 26A and 26B** show exemplary paths of current when stimulation between a pair of electrodes connected to the electrode terminals E1 and E2 in the first circuit C0 is performed. In this example, the first, fourth, fifth and sixth switches S01, S04, S05, S06 of the first circuit C0 are set to the closed state and the remaining switches S02, S03 of the first circuit C0 are set to the open state. The first circuit C0 may be understood as operating in the stimulation mode as shown in Table 2B. In the second circuit C1, the fifth and sixth switches S15, S16 are set to the closed state and all other switches S11, S12, S13, S14 are set to the open state. The second circuit C1 may be understood as operating in the inactive mode as shown in Table 2B. In the example of Fig. 26B where the first and second circuits C0, C1 respectively include the ground connecting switches SG0, SG1, the ground connecting switches SG0 and SG1 may be set to the closed state.

In the examples of Figs. 26A and 26B, the stimulation current may flow from a current source that is connected to the power terminal PT0 of the first circuit C0 and that generates the current signal $i_{pattern1}$. The stimulation current may then follow a path including the first switch S01, the electrode terminal E1, the electrode terminal E2, the fourth switch S04, the sixth switch S06, the resistance $R_{M0}$ and the ground terminal GT0, as shown with the arrows of thick solid lines in Figs. 26A and 26B. In these examples, the current source generating the current signal $i_{pattern2}$ is made inactive by setting the switches of the second circuit C1 as the inactive mode, thereby realizing virtual galvanic isolation.

**Figs. 27A and 27B** show exemplary paths of current when stimulation between a pair of electrodes connected to the electrode terminal E1 of the first circuit C0 and the electrode terminal E4 of the second circuit C1 is performed. In this example, the stimulation current is desired to flow in a direction from the electrode terminal E4 to the electrode terminal E1. Accordingly, the second switch S12 of the second circuit C1 is set to the closed state for electrically connecting the electrode terminal E4 to the power terminal PT1 of the second circuit C1 so that the current signal $i_{pattern2}$ can flow into the electrode terminal E4. In the second circuit C1, the first, third and fourth switches S11, S13, S14 are set to the open state so that the electrode terminal E3 is electrically disconnected from the power terminal PT1 and the ground terminal GT1 and that the electrode terminal E4 is electrically disconnected from the ground terminal GT1. Further in the second circuit C1, the fifth and sixth switches S15, S16 are set to the closed state for protecting the first differential amplifier A11. In the first circuit C0, on the other hand, the first, second and fourth switches S01, S02, S04 are set to the open state, electrically disconnecting the both electrode terminals E1, E2 from the power terminal PT0 and further electrically disconnecting the electrode terminal E2 from the ground terminal GT0. The third and fifth switches S03, S05 of the first circuit C0 are set to the closed state so that the electrode terminal E1 is electrically connected to the ground terminal GT0. The sixth switch S06 is also set to the closed state in order to protect the first differential amplifier A01. In the example of Fig. 27B where the first and second circuits C0, C1 respectively include the ground connecting switches SG0, SG1, the ground connecting switches SG0 and SG1 may be set to the closed state.

In the examples of Figs. 27A and 27B, the stimulation current may flow from the current source connected to the power terminal PT1 of the second circuit C1. The stimulation current may first flow into the electrode terminal E4 via the first switch S12 of the second circuit C1. The stimulation current may further flow to the electrode connected to the electrode terminal E4, then to the skin of the human or animal subject on which the electrodes are applied and reach the electrode connected to the electrode terminal E1 of the first circuit C0. Then the stimulation current may follow the path including the electrode terminal E1, the third switch S03, the fifth switch S05, the resistance $R_{M0}$ and the ground terminal GT0 of the first circuit C0. The exemplary current paths as stated above are indicated in Figs. 27A and 27B with arrows with thick solid lines and a thick broken line connecting the electrode terminals E1 and E4.

Similarly to the cases of Embodiments 1 and 2, also in this Embodiment 3, stimulation can be made by a pair of electrodes connected to any arbitrary pair of electrode terminals selected from the electrode terminals in the first and second circuits C0, C1 (e.g., any pair selected from electrode terminals E1 to E4). The switch control performed by the controller 20 as explained above in connection with Embodiment 1 and Figs. 10 to 12 may be applicable analogously for controlling, during the stimulation mode, the medical device 10 including circuits 12 each of which has the exemplary configuration shown in Fig. 21. With such a medical device 10, however, during the stimulation mode, the fifth and sixth switches of each circuit 12 may be set to the closed state for protecting the first differential amplifier A1.

**Figs. 28A and 28B** show exemplary paths of current when impedance measurement between a pair of electrodes connected to the electrode terminal E1 of the first circuit C0 and the electrode terminal E4 of the second circuit C1 is performed. In this example, current for the impedance measurement is desired to flow in a direction from the electrode terminal E4 to the electrode terminal E1. Accordingly, the second switch S12 of the second circuit C1 is set to the closed state so as to electrically connect the electrode terminal E4 with the power terminal PT1, enabling the electrode terminal E4 to receive the current signal $i_{pattern2}$ from the current source. The first and third switches S11, S13 of the second circuit C1 are set to the open state so that the electrode terminal E3 is electrically disconnected from the power terminal PT1 and the ground terminal GT1. Further in the second circuit C1, the fourth and fifth switches S14, S15 are set to the closed state and the sixth switch S16 is set to the open state. Such switch settings of the second circuit C1 make the potential of the measurement terminal M3 equal to (or almost equal to with a negligible error) the ground potential and the potential of the measurement terminal M4 equal to (or almost equal to with a negligible error) that of the electrode terminal E4. Consequently, the output of the first differential amplifier A11 of the second circuit C1 may be an amplified value of the voltage between the electrode terminal E4 and the ground, which may be represented as $V_{E4-GND}$.

In the first circuit C0 shown in Figs. 28A and 28B, the first and second switches S01, S02 are set to the open state so that the electrode terminals E1, E2 are electrically disconnected from the power terminal PT0. The third and fifth switches S03, S05 are set to the closed state so as to electrically connect the electrode terminal E1 to the ground terminal GT0. Further, the fourth switch S04 is set to the open state for electrically disconnecting the electrode terminal E2 from the ground terminal GT0 and the sixth switch S06 is set to the closed state so that the first differential amplifier A01 may be protected. Such switch settings of the first circuit C0 make the potential of the measurement terminal M1 equal to (or almost equal to with a negligible error) the potential of the electrode terminal E1. Consequently, the output of the second differential amplifier A02 of the first circuit C0 may be an amplified value of the voltage between the electrode terminal E1 and the ground, which may be represented as $V_{E1-GND}$.

In the example of Fig. 28B where the first and second circuits C0, C1 respectively include the ground connecting switches SG0, SG1, the ground connecting switches SG0 and SG1 may be set to the closed state.

The current for impedance measurement may flow through a path indicated in Fig. 28 by arrows with thick solid lines and a thick dashed line connecting the electrode terminals E4 and E1. The path indicated in Fig. 28 includes, in the order of the current flows, the power terminal PT1, the second switch S12 and the electrode terminal E4 of the second circuit C1 as well as the electrode terminal E1, the third and fifth switches S03, S05, the resistance $R_{M0}$ and the ground terminal GT0. The current flows from the electrode terminal E4 to the electrode terminal E1 via a pair of electrode connected to the electrode terminals E4 and E1 and the skin of the human or animal subject to which the electrodes are applied.

As stated above, in the examples of Figs. 28A and 28B, the output of the first amplifier A11 of the second circuit C1 may be an amplified value of the voltage $V_{E4-GND}$ between the electrode terminal E4 and the ground and the output of the second amplifier A02 of the first circuit C0 may be an amplified value of the voltage $V_{E1-GND}$ between the electrode terminal E1 and the ground. Thus, the voltage $V_{E4-E1}$ between the electrode terminals E4 and E1 may be obtained by subtracting $V_{E1-GND}$ from $V_{E4-GND}$. Further, the current $i_{pattern2}$ flowing through the paths shown in Figs. 28A and 28B may be obtained using the known value of the resistance $R_{M0}$ and the output of the second differential amplifier A02 of the first circuit C0. From the current $i_{pattern2}$ thus obtained and the voltage $V_{E4-E1}$ between the electrode terminals E4 and E1, the impedance between the pair of electrodes connected to the electrode terminals E4 and E1 may be determined.

Figs. 28A and 28B shows merely one example of the current path and switch settings for impedance measurement using the medical device 10. As in the case of stimulation, impedance measurement can be made for a pair of electrodes connected to any arbitrary pair of electrode terminals selected from the electrode terminals in the first and second circuits C0, C1 (e.g., any pair selected from electrode terminals E1 to E4).

In case the selected pair of electrode terminals belong to the same circuit, the controller 20 may control the medical device 10 to set each switch of the circuit including the selected pair of electrode terminals as shown in the impedance measurement mode of Table 2B. Further, in this case, the controller 20 may control the medical device 10 to set each switch of the circuit not including the selected pair of electrode terminals as shown in the inactive mode of Table 2B.

In case each one of the selected pair of electrode terminals is comprised in a different circuit, the controller 20 may control the medical device 10 to set each switch in the first and second circuits C0, C1 to the closed state or the open state so as to:

- electrically connect one of the selected pair of electrode terminals to the power terminal PT and the first or second measurement terminal of the circuit comprising the one of the selected pair of electrode terminals;
- electrically connect, to the ground terminal GT via the resistance $R_M$ (and the ground connecting switch SG, where applicable), the first or second measurement terminal that is comprised in the circuit comprising the one of the selected pair of electrode terminals and that is not electrically connected to the one of the selected pair of electrode terminals;
- electrically connect another one of the selected pair of electrode terminals to the first or second measurement terminal of the circuit comprising the other one of the selected pair of electrode terminals;

- electrically connect, to the ground terminal GT via the resistance $R_M$ (and the ground connecting switch SG, where applicable), the first and second measurement terminals of the circuit comprising the other one of the selected pair of electrode terminals; and
- electrically disconnect the first and second electrode terminals other than the selected pair of electrode terminals from the ground terminal GT and from the power terminal PT.

According to the control of switch settings as stated above, the current for impedance measurement may flow from the power source to one of the selected pair of electrode terminals that is electrically connected to the power terminal PT (thus to the power source), and then, via the pair of electrodes applied to a human or animal subject, to the other one of the selected pair of electrode terminals that is electrically connected to the ground terminal GT (thus to the ground). Further, according to the control of switch settings as stated above, the voltage between the selected pair of electrode terminals may be determined from the output of the first differential amplifier connected to one of the selected pair of electrode terminals that receive power from the power source and from the output of the second differential amplifier connected to the other one of the selected pair of electrode terminals that is electrically connected to the ground terminal GT via the resistance $R_M$. The above-stated control of switch settings may be applicable also to the medical device 10 that has three or more circuits 12.

Although the above explanation on the exemplary current paths with reference to Figs. 26A to 28B relate to the medical device 10 having more than one circuits 12 receiving input power from different power sources, the same may apply to the medical device 10 having more than one circuits 12 receiving input power from a single power source.

< Advantages >

The various exemplary configurations of the circuit(s) 12 included in the medical device 10 as described above in this Embodiment 3 (see e.g. Figs. 20, 21, 23, 24 and 25) may enable electric stimulation as well as voltage, current and impedance measurements. The voltage measurement may be applied to biosignal measurements, for example, EMG, ECG, EOG, etc. The impedance measurement may be applied to measurement of tissue or dermal resistance, e.g. EDR.

Since the stimulation as well as voltage, current and impedance measurement may be performed with respect to a pair of electrodes connected to any arbitrary pair of selected electrode terminals of the circuits 12, the various exemplary configurations described above in this Embodiment 3 provides great flexibility in the operation of the medical device 10 and the interconnection of the electrodes.

As in Embodiments 1 and 2, the medical device 10 including more than one circuits 12 with the exemplary configuration of Fig. 20, 21 or 25A do not need true galvanic isolation when appropriate switch control is performed. Specifically, by controlling the switches of the circuits 12 so that only one power source channel is made active for stimulation, virtual galvanic isolation between circuits 12 receiving power from different power sources may be achieved.

Further, the various exemplary configuration of the circuits 12 as shown in Figs. 20, 21, 23, 24 and 25A may enable measurement of currently flowing current within the circuit(s) 12, which may then enable control of the strength of the current signal and adjustment thereof when required.

The biosignal measurement function of the medical device 10 may be improved by employing one or more circuit 12 having the exemplary configuration shown in Fig. 21 or 25A as compared to the case of using the circuit 12 shown in Fig. 20. Since the input impedance of the first differential amplifier A1 of the exemplary circuit 12 shown in Fig. 21 or 25A can be made higher than that shown in Fig. 20 by employing a differential amplifier with lower voltage tolerance, the accuracy of the biosignal measurement may be improved.

With the medical device 10 including the circuit(s) 12 having the exemplary configuration shown in Fig. 25A and 25B with the ground connecting switch SG, the virtual galvanic isolation may be realized during the short circuit mode. The short circuit mode may contribute to the recovery of the stimulated part of the skin, as stated above with respect to the advantages of Embodiments 1 and 2.

## Embodiment 4: Circuits with True Galvanic Isolation

In the above Embodiments 1 to 3, more than one circuits 12 of the medical device 10 receiving input power from more than one power sources may have virtual galvanic isolation by activating only one power source channel at a time with appropriate control of the switch settings. Thus, the circuits 12 and the power sources do not need to have true galvanic isolation in these examples.

In the present Embodiment 4, however, the circuits 12 and the power sources may have true galvanic isolation. In other words, the circuits 12 receiving input power from different power sources may be electrically isolated using an actual galvanic isolation technique. In such examples, each circuit 12 of the medical device 10 may have its own input voltage $V_H$ and the ground GND that are electrically isolated from the input voltage $V_H$ and the ground GND for other circuits 12. The exemplary configurations of the medical device 10 of Embodiment 4 may appear similar to those shown in Figs. 5, 17 and 23, except that the circuits 12 would not share the input voltage $V_H$ and the ground GND but rather have respective input voltages $V_H$ and the grounds GND with true galvanic isolation.

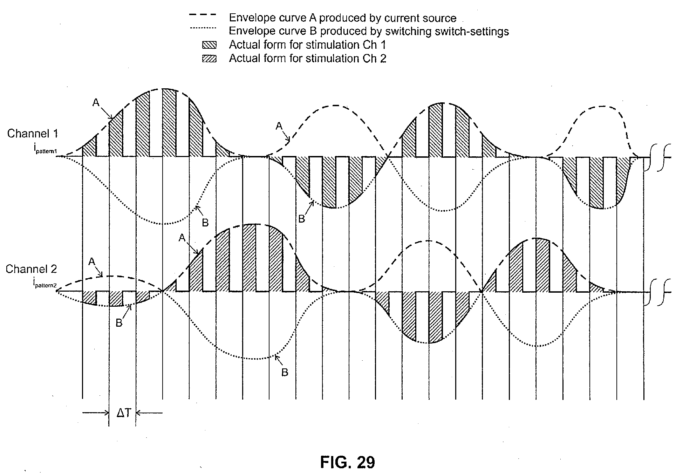

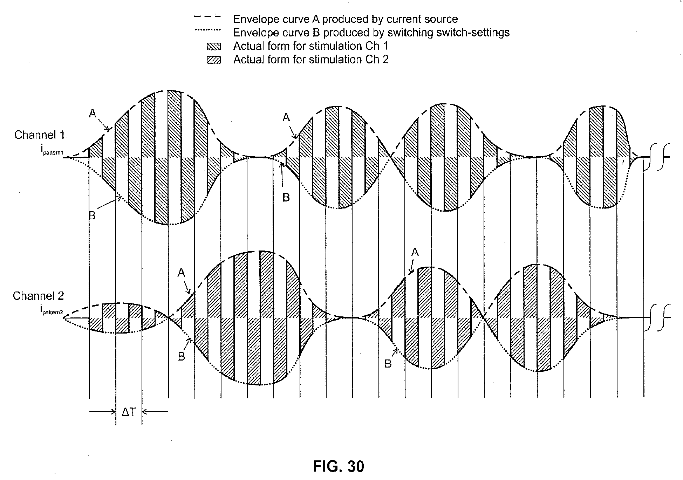

When the circuits 12 have true galvanic isolation, more than one power source channels may be activated at the same time and stimulation using the first and second electrode terminals in different circuits 12 may be performed at the same time. Accordingly, exemplary signal patterns of the current signals applied to the circuits 12 of the medical device 10 with true galvanic isolation may appear as shown in **Figs. 29 and 30.**

**Fig. 29** shows exemplary signal patterns in case of LF stimulation and **Fig. 30** shows exemplary signal patterns in case of MF stimulation. In Figs. 29 and 30, signal patterns of two current sources for channels 1 and 2 are shown. Further, in Figs. 29 and 30, the curves with dashed and dotted lines of the current signals $i_{pattern1}$ and $i_{pattern2}$ represent the envelope curves generated by respective current sources and switch-settings and parts of the envelope curves during time periods indicated by the hatched areas represent the actual signal forms used for stimulation. In Fig. 30, the curve with the dotted line B represents a current signal having an opposite direction from the current signal represented by the curve with the dashed line A. The change of directions of the current signal may be realized by changing the settings of the switches of the circuit 12, as described above with respect to the Embodiments 1 to 3. As can be seen from Figs. 29 and 30, when the circuits 12 receiving power from different power sources have true galvanic isolation, two power source channels may be activated at the same time.

< Advantages >

The medical device 10 including circuits 12 with true galvanic isolation can provide secure electric isolation of the human or animal subject. Further, since each circuit 12 and the relevant power source can be electrically isolated from other circuits 12 and power sources, adding one or more circuits 12 for extension to enable more electrodes to be available can easily be made. Moreover, the switch control during operation can be made simpler since it is not necessary to ensure that only one power source channel is made active at a time. In other words, different stimulation current signals for different circuits 12 may be used at the same time. Further, even with a low frequency stimulation signal $i_{pattern}$, by modulation (oversampling with switch control), stimulation with a high frequency may be possible, which may allow a larger penetration depth of the current flows into the tissues of the human or animal subject.

The above stated advantages of the medical device 10 with respect to the Embodiments 1 to 4 may be especially beneficial when the medical device 10 is implemented as a portable device, concerning the size, weight, battery consumption and price, for example.

**Floating Current Source**

The power source that provides input power to the exemplary circuits 12, C0, C1 of the Embodiments 1 to 4 as stated above may be a floating current source.

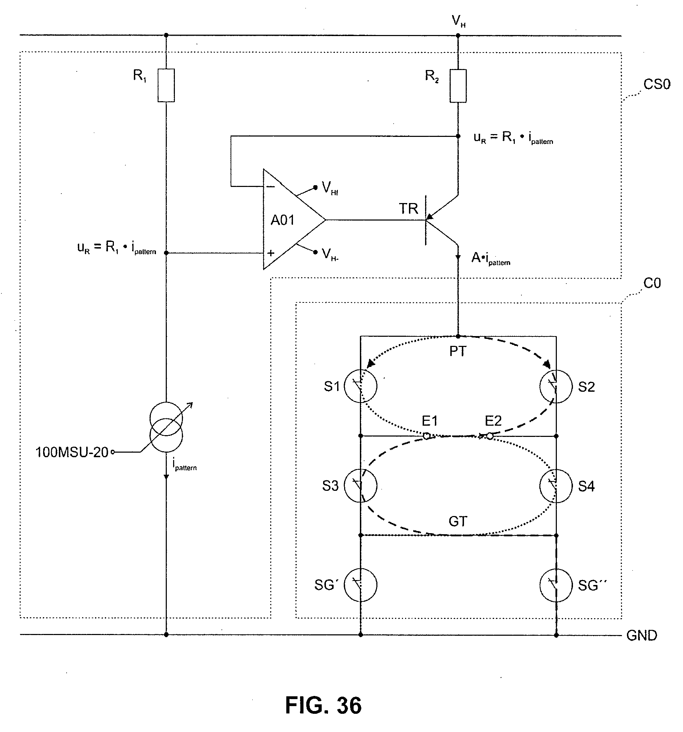

**Fig. 36** shows an exemplary implementation of the circuit C0 and a possible implementation of the signal current source CS0 as a floating current source in accordance with Fig. 15.

In Fig. 36, the collector of a PNP transistor TR is connected to the power terminal PT. The basis of the PNP transistor TR is connected to the output of an operation amplifier A01. The emitter of the PNP transistor TR is connected to the minus input of operation amplifier A01 and one end of a resistor $R_2$. The other end of the resistor $R_2$ is connected to a terminal $V_H$. Also connected on the terminal $V_H$ is one end of the resistor $R_1$. The other end of the resistor $R_1$ is connected to the plus input of the operation amplifier A01 and one end of a voltage-controlled constant current source $i_{pattern}$. The other end of the voltage-controlled constant current source $i_{pattern}$ is connected to the ground GND. The regulation input voltage for the voltage-controlled constant current source $i_{pattern}$ comes from a microcontroller 100MSU-20. The positive supply voltage $V_{Hf}$ of the operation amplifier A01 is a low-pass filtered voltage of $V_H$. The negative supply voltage $V_{H-}$ of the operation amplifier A01 is a stabilized lower voltage formed from $V_H$.

The microcontroller 100MSU-20 may generate a voltage, for example, by means of a digital-to-analog converter. This voltage may control the voltage-controlled constant current source $i_{pattern}$. Here, the generated voltage may be converted into a proportional current. The current $i_{pattern}$ through the resistor $R_1$ may enforce a voltage drop $u_R = R_1 \cdot i_{pattern}$ across the resistor $R_1$. No current or negligibly low current may flow into the minus- and plus-inputs of the operation amplifier A01. The operation amplifier A01 may try to reach a voltage difference of 0V between the minus- and plus-inputs thereof. Therefore, the operation amplifier A01 may regulate the PNP-transistor TR such that a voltage drop across the resistor $R_2$ will be $u_R = R_1 \cdot i_{pattern}$.

The voltage terminal $V_H$ can be e.g. strongly wavy and fluctuating due to the voltage generation. The fluctuations of $V_H$ may not influence the stimulation current for the circuit C0, because of the operation-amplifier-PNP-transistor regulation and the creation of the voltage $u_R$. The PNP transistor TR may amplify the current $i_{pattern}$ by a factor A, to A $\cdot i_{pattern}$ and let the current A $\cdot i_{pattern}$ flow to the circuit C0.

If the circuit C0 is, for example, in the stimulation mode and the switches S1, S4, SG' and SG" are set to the closed state and the other switches S2 and S3 are set to the open state, the current A • $i_{pattern}$ can flow from the electrode terminal E1 through a human or animal subject (e.g. a patient P) to the electrode terminal E2 and then to the ground GND. With this switch settings and the current A • $i_{pattern}$ may create the envelope curve A shown in Fig. 19. Further, if the switches S2, S3, SG' and SG" are set to the closed state and the switches S1 and S4 are set to the open state, the envelope curve B shown in Fig. 19 may be created.

If both switches S1 and S2 are set to the open state and therefore non-conductive, the whole circuit C0 may be nearly infinitely high impedance for the collector current A • $i_{pattern}$ of the PNP-transistor TR. During the switching process of the circuit C0, the collector-emitter-voltage $u_{CE}$ may become smaller and smaller until the transistor is strangulated. No collector current can then flow through the circuit C0. The current from the voltage terminal $V_H$ across the resistor $R_2$, forced by the voltage $u_R$, may flow away in the operations amplifier output.

If the circuit C0 is "conductive", for example, in the stimulation mode (e.g. in case the switches S1, S4, SG' and SG" are set to the closed state and switches S2 and S3 are set to the open state), since the transistor may have been prestressed by the operation amplifier A01, an overdrive current may flow at first in the circuit C0 for a very short period of time, e.g. a few microseconds.

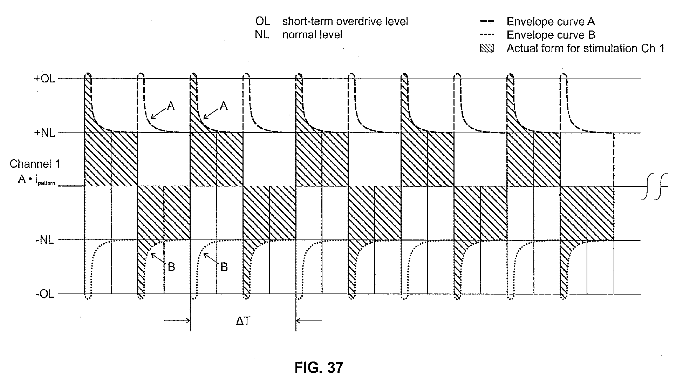

**Fig. 37** shows an exemplary signal pattern in case of MF stimulation using the circuit(s) 12 with the ground connecting switch(es) SG and the floating current source CS0 as shown in Fig. 36. The dashed line in Fig. 37 represents the positive envelope curve A of the current generated by the floating current source CS0. The dotted line in Fig. 37 represents the envelope curve B also generated by floating current source CS0 but in another flow direction realized by changing switch-settings in the circuit C0 in a way analogous to that as explained above in connection with the Embodiment 1. The hatched areas shown in Fig. 37 indicate the actual signal forms used for stimulation between electrodes connected to the electrode terminals E1 and E2. The thin solid lines are grid-lines. The current level NL represents the desired and actual current level set by the microcontroller 100MSU-20. The current level OL represents an overdrive current that flows for only a very short period of time, with regards to its limited amplitudes. In most electronics circuits, an "overshoot" or overdrive of a current signal is not desired, but in case of muscle and nerve stimulation, it may be beneficial. This overdrive current may provide a stronger activation of muscles and nerves. Because of its very short duration (e.g., in the range of microseconds) and its still limited amplitudes, the overdrive current may not be dangerous for a human or animal subject.

The overdrive current may be obtained safely and inexpensively with the switch configurations of the circuits C0, C1 as described above, with generation of a positive current by the PNP-transistor TR and with realizing changes of the current flow directions by changing the switch settings as described above.

If the circuit C0 is non-conductive (e.g. the switches S1 and S2 are set to the open state), the PNP-transistor TR may not have a ground reference anymore. Further, the voltage terminal $V_H$ may fluctuate. The lack of a ground reference for the PNP-transistor TR and the fluctuation of the voltage terminal $V_H$, however, may not influence the current source $i_{pattern}$. Accordingly, the current source $i_{pattern}$ as can be realized by the exemplary configuration shown in Fig. 36 may be called a "floating current source".

A floating current source having the exemplary configuration shown in Fig. 36 may be employed as the power source for any one of the circuits 12, C0, C1 of the Embodiments 1 to 4 described above.

**Medical Device 100 for Influencing a Breathing Condition of a Patient P**

In the following, there is described with reference to **Figs. 31-33** a medical device 100 being used for influencing the breathing or a breathing condition of a human patient P in a situation of partly obstructed or restricted respiratory ducts (hypopnea) and/or completely or fully obstructed respiratory ducts (apnea), specifically occurring during sleep apnea (also referred to as sleep apnea syndrome). Specifically, the described medical device 100 being used for influencing the breathing or a breathing condition of a human patient P may be implemented using the above described medical device 10 and specifically the circuit configuration used therein.

**Fig. 31** shows different breathing situations of a patient P. In **Fig. 31A-1** there is shown a normal breathing situation of the patient P, where respiratory ducts of the patient P are substantially completely open. A tongue P30 of the patient P touches a palate P40 so that air can flow through a nasal cavity P50 through a buccal cavity P20 and an airway or trachea P10 of the patient P. In such a situation, an aperture N of the respiratory ducts is substantially normal. Accordingly, an airflow B-N of the patient P shown in **Fig. 31A-2** corresponds to a normal breathing situation.

In **Fig. 31B-1** is shown a restricted breathing situation of the patient P, where respiratory ducts of the patient P are restricted R. Specifically, the air passageway between the buccal cavity P20 and the trachea P10 corresponds to a reduced or restricted aperture R, leading to the restricted breathing situation (referred to as hypopnea or obstructive hypopnea), where an airflow B-R of the patient P is at least temporarily reduced (i.e. has a reduced amplitude) with respect to the normal airflow B-N (see **Fig. 31A-2**).

**Fig. 31C-1** specifically shows an obstructed breathing situation where respiratory ducts of the patient P are fully obstructed, thus, leading to an obstructed aperture O which substantially does not allow any air to flow towards the trachea P10. Accordingly, as shown in **Fig. 31C-2,** an airflow B-O of the patient P is (at least temporarily) completely interrupted, corresponding to the obstructed breathing situation (also referred to as apnea).

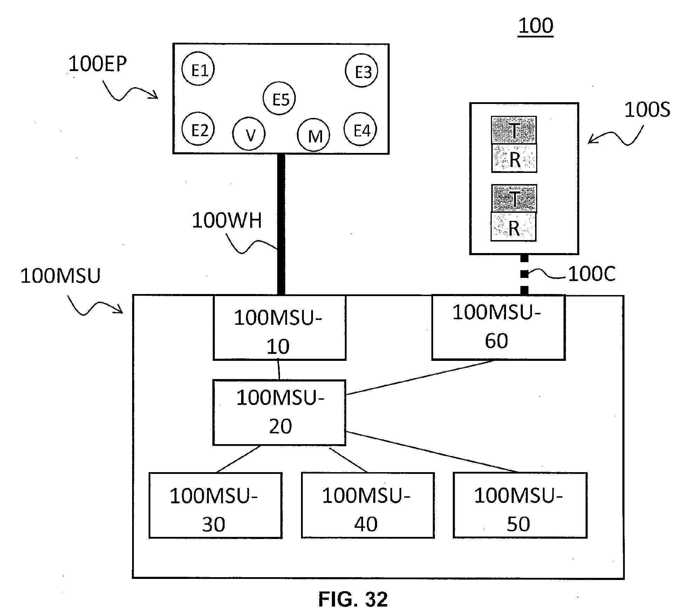

A basic setup of an embodiment of the medical device 100 for influencing or controlling the breathing of the patient P is shown in **Fig. 32.** In this embodiment, the medical device 100 comprises at least one electrode patch 100EP to be applied to the patient P, as described hereinafter. The electrode patch 100EP may comprise two or more electrodes E1, E2, E3, E4, E5, etc. which are to be placed (particularly fixed) on or in the skin of the patient P. The electrode patch 100EP may further comprise at least one microphone M for sensing or detecting noises and/or at least one vibration sensing device V for sensing or detecting vibrations or vibration patterns generated by the patient P. The electrode patch 100EP may be particularly configured as an adhesive patch which can be placed and secured to the patient P by means of an adhesive layer at least partly provided thereon. Alternatively or additionally, the electrode patch 100EP may comprise one or more securing members (such as one or more straps) to fixedly secure the electrode patch 100EP to the patient P in a specified (predetermined or predeterminable) position, as particularly described hereinafter.

The medical device 100 of this embodiment further comprises a measurement and/or stimulation unit 100MSU, which is to be signal connected to the electrode patch 100EP particularly via a wired connection 100WH (such as a wire harness). It should be understood, however, that any other signal connection, particularly wireless signal connection between the measurement and/or stimulation unit 100MSU and the electrode patch 100EP is also possible according to the present embodiment. The unit 100MSU particularly comprises an analog measurement and/or stimulation module (specifically analog measurement and/or stimulation circuitry) 100MSU-10, as a particular control unit, capable of receiving signals or measuring signals received from the electrode(s) E1-E5 and/or the microphone M and/or the vibration sensing device V of the electrode patch 100EP and/or applying one or more stimulation signals to the electrode(s) E1-E5 of the electrode patch 100EP. Specifically, the module 100MSU-10 may be embodied by an I/O interface 34a as described above. It should be understood that the module 100MSU-10 may also be (at least partly) embodied in a digital manner according to the present embodiment, such as by providing analog analog-to-digital converters (ADC) and/or by directly receiving/transmitting at least part of the signals in a digital manner from/to the electrode patch 100EP. The electrode patch 100EP and its respective control by the measurement and/or stimulation unit 100MSU may particularly be embodied by circuit(s) and/or the controller 20 and medical device 10 as described above with reference to Figs. 2 to 5 and 11, 12, 14 to 17, 20 to 24 and/or 26 to 28.

The measurement and/or stimulation unit 100MSU further comprises a microcontroller 100MSU-20 which is signal connected to the measurement and/or stimulation module 100MSU-10. Specifically, the measurement and/or stimulation unit 100MSU may be (partly) embodied by means of a controller 20 as described above. The microcontroller 100MSU-20 particularly analyses the one or more signals received by the measurement and/or stimulation unit 100MSU-10 by comparing them with one or more reference signals stored in a database 100MSU-30. Furthermore, the microcontroller 100MSU-20 may store at least part of the measured signals in the database 100MSU-30. The database 100MSU-30 may be particularly embodied by the hard disk drive (HDD) 28 of the controller 28.

The measurement and/or stimulation unit 100MSU may further comprise at least one input/output module 100MSU-40, particularly including at least one display and/or at least one I/O interface, and/or a power storage and management module 100MSU-50 to provide current to the measurement and/or stimulation unit 100MSU and/or to the electrode patch 100EP via the connection 100WH. Specifically, the input/output module 100MSU-40 may be embodied by means of the I/O interfaces 34b, 34c described above.

The measurement and/or stimulation unit 100MSU may further comprise a communication module 100MSU-60 being connected to the microcontroller 100MSU-20 and able to communicate with at least one sensor element 100S, being separately provided from the measurement and/or stimulation unit 100MSU. Specifically, the communication module 100MSU-60 may be embodied by an I/O interface 34a as described above. Specifically, the sensor element 100S may communicate with the communication module 100MSU-60 via wireless communication (e.g. Bluetooth, WLAN, or the like).

The sensor element 100S comprises one or more transmitter elements T and/or one or more receiver elements R and is to be placed on or arranged at the patient P to detect one or more biosignals or medical conditions or parameters (such as blood pressure, pulse amplitude, pulse-transit-time (PTT) and the like) to be described hereinafter. The microcontroller 100MSU-20 further particularly analyses the one or more signals received from the sensor element 100S via the communication module 100MSU-60 particularly by comparing them with one or more reference signals stored in a database 100MSU-30. Furthermore, the microcontroller 100MSU-20 may store at least part of the received signals in the database 100MSU-30. The sensor element 100S particularly is arranged on the wire connection 100WH and the connection 100C further specifically may be included into the wire connection 100WH. The microcontroller 100MSU-20 may use the signal received from the microphone M and/or vibration sensor V provided at the electrode patch 100EP to determine the breathing condition of the patient P, e.g. by detecting any snoring activity of the patient P, e.g. by a sound level of the microphone M being higher than a specified (predetermined or predeterminable) threshold level. In noisy environments, such as an aircraft cabin, a control based on a threshold level may not be sufficient, so that in such cases the spectrum, the waveform and/or the level of the sound may be analyzed.

Any signal analysis result and/or corresponding signal(s) received from the electrode patch 100EP and/or sensor element 100S may be (particularly selectively) output by the microcontroller 100MSU-20 to an operator via the input/output module 100MSU-40 e.g. via a graphical user interface. The microcontroller 100MSU-20 may control the measurement and/or stimulation module 100MSU-20 such as to apply one or more stimuli to the patient P via the electrode(s) E1-E5 of the electrode patch 100EP. Specifically, the application of one or more stimuli may only occur when an operator has given a corresponding input (e.g. validation and/or setting of one or more specific control parameters) via the I/O interface of the input/output module 100MSU-40.

Accordingly, the measurement and/or stimulation unit 100MSU is able to (particularly continuously) monitor the breathing condition of the patient P via the electrode patch 100EP and/or the sensor element 100S and intervene in case it detects or predicts an anomalous situation such as an apnea or hypopnea. In other words, the medical device 100 can monitor a breathing activity of the patient P by means of the electrode patch 100EP and/or sensing element 100S and detect and/or predict a specified (predetermined or predeterminable) breathing situation or condition of the patient P which triggers a stimulation of the patient P by applying one or more electric stimuli to the patient P via the electrode patch 100EP.

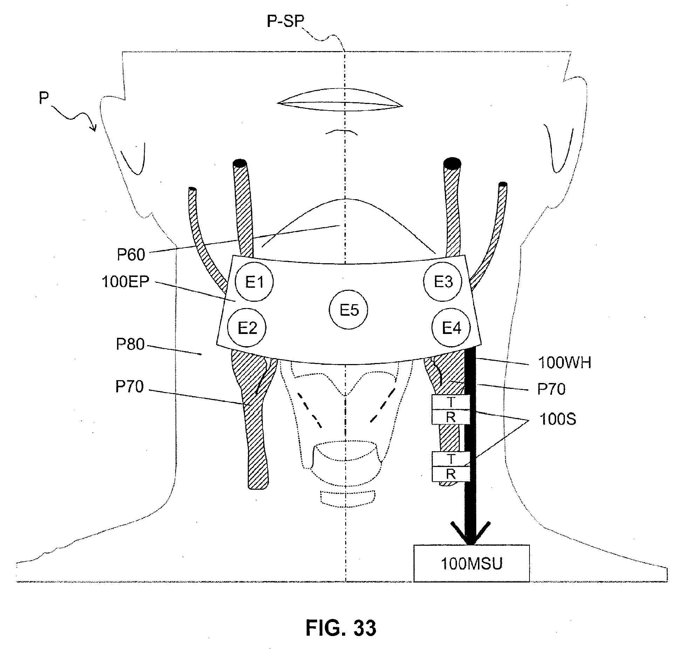

A specified (predetermined or predeterminable) arrangement of the at least one electrode patch 100EP on the patient P is described in reference to **Fig. 33.** The electrode patch 100EP is to be place (directly) on the skin of the patient P in proximity and below of a chin P60 of the patient P such that at least one pair of electrodes E1, E2 are arranged adjacent to the musculus genioglossus (GG) of the patient P. Specifically, the electrode patch 100EP is to be placed substantially symmetric on a throat P80 of the patient P. A further electrode E5 is to be placed substantially centrally (or on a sagittal plane P-SP of the patient P) on the throat P80 of the patient P and serves as the reference potential for the patient P ($GND_{Patient}$). Particularly, the reference potential for the patient P ($GND_{Patient}$) may be used as the reference for the measurement module A1 of the medical device 10 described above. At least one electrode pair E1/E2 (and/or E3/E4) is to be placed on or arranged at or in proximity of the nervus hypoglossus of the patient P so as to be able to stimulate the nervus hypoglossus in an event of an abnormal breathing condition of the patient P determined by the measurement and/or stimulation unit 100MSU. The electrode(s) E1-E5 particularly are in direct contact with the skin of the patient P and are able to apply one or more stimuli to a tissue of the patient P particularly by electric power (or electric current(s) and/or voltage(s)) applied thereto.

Specifically, it in this embodiment, the electrode patch 100EP has at least five electrodes E1 to E5. A first pair of electrodes E1/E2 and a second pair of electrodes E3/E4 are arranged substantially along the musculus genioglossus (GG) or in proximity of the nervus hypoglossus particularly in a substantially axially symmetrical relationship with respect to the sagittal plane P-SP of the patient P, in order to be able to stimulate the nervus hypoglossus, particularly through the skin of the patient P, in case an abnormal breathing condition (e.g. apnea or hypopnea) is determined or predicted by the measurement and/or stimulation unit 100MSU.

One or more sensor elements 100S may be arranged on or near the arteria carotis P70 to particularly detect the pulse-transit-time (PTT), the blood pressure, the hearth frequency, the plethysmograph pulse amplitude of the patient P. Specifically, the sensor element(s) 100S are physically arranged on the wire connection 100WH and/or can be fixed to a throat area of the patient P on or near the arteria carotis P70.

The one or more signals detected or sensed by the electrode patch 100EP and/or the sensor element 100S are analysed by the microcontroller 100MSU-20 of the measurement and/or stimulation unit 100MSU. The abnormal breathing condition (such as hypopnea or apnea) of the patient P comes along with a modification of several of the patient's biosignals (such as electroencephalography (EEG), impedance change around the trachea, the pulse-transit-time (PTT), the blood pressure, the hearth frequency, electromyogram (EMG), electrocardiogram (ECG, EKG), the electrodermal response (EDR), the pulse amplitude and/or the breathing frequency). Accordingly, by detecting and analyzing respective one or more biosignals (e.g. at or near the arteria carotis P70) a (potentially) abnormal breathing condition can be detected or predicted. Moreover, the abnormal breathing condition can be detected or predicted by the microcontroller 100MSU-20 based on the signal(s) received from the microphone M and/or by the vibration sensing device V. Particularly, the signals received from the microphone M and/or by the vibration sensing device V may be used to confirm a prediction or detection of the abnormal breathing situation based on the signals received from the electrodes E1-E5 of the electrode patch 100EP and/or from the sensing element 100S. For the purpose of detecting or predicting the abnormal breathing condition, the microcontroller 100MSU-20 applies suitable multivariant methods which are utilized to detect significant features distinguishing a normal breathing condition (i.e. without hypopnea or apnea) from the abnormal breathing condition (i.e. with hypopnea or apnea). It is also possible to implement suitable learning algorithms to allow a more accurate prediction/detection of an abnormal breathing condition of the patient P. Advantageously, the signal detection and/or abnormal breathing condition prediction/detection are active, when the electrode patch 100EP is applied to the patient P. If an abnormal breathing condition is determined or predicted by the measurement and/or stimulation unit 100MSU e.g. using the reference data stored in the database 100MSU-30 at least one pair of electrodes E1/E2 and/or E3/E4 can be used to transcutanely (directly or indirectly) stimulate the nervus hypoglossus and/or the musculus genioglossus to influence the breathing condition.

It should be understood that the stimulation of the nervus hypoglossus and/or the musculus genioglossus may be achieved by means of one or more electrodes being at least partly inserted into the skin and/or tissue of the patient P. For this purpose, one or more electrodes E1 to E5 may be configured as pointed electrode(s) being able to be at least partly inserted into the patient's skin.

By stimulating the nervus hypoglossus and/or the musculus genioglossus, the tongue P30 of the patient P is caused to move in the direction toward the patient's lips, thus reducing or cancelling the obstruction of the patient's airway. In other words, by stimulating the nervus hypoglossus and/or the musculus genioglossus the obstructed aperture O (Fig. 31C-1) can be actively positively influenced (enlarged) to become at least a restricted aperture R (Fig. 31B-1) or (possibly) to substantially a normal aperture N (Fig. 31A-1) leading at least to a restricted breathing airflow B-R or (possibly) to a normal breathing airflow B-N and/or the restricted aperture R can be actively influenced to substantially become a normal aperture N (Fig. 31A-1) leading to a normal breathing airflow B-N.

Specifically, the current paths can be generated between any one of the electrodes E1-E4 in a similar way as described with reference to Fig. 13. Specifically, it is possible to generate a current between external electrodes in the following manner: E1 → E2, E3 → E4, E1 → E3, E2 → E4, and/or E1 → E4 (wherein Ei -> Ej denotes the current flow through the patient P from the electrode Ei to the electrode Ej). The stimulation shape (i.e. the current or signal pattern), e.g. the middle frequency (MF) stimulation described above with respect to Fig. 8, 9 or 19, which is applied to the respective electrodes E1-E4, can be adapted to the anatomic and/or physiologic circumstances of each patient P or of respective groups of patients P. In this particular embodiment, the medical device 100 may be embodied by means of a medical device 10 described above with reference to Figs. 1 to 30, which particularly comprises a first circuit 12, C0 for selectively applying power to at least two electrodes applied to a human or animal subject, the first circuit 12, C0 including: a first switch S1 electrically connected to a power terminal PT and a first electrode terminal E1, the power terminal PT being configured to receive input from a power source, the first electrode terminal E1 being configured to be electrically connected to one of said at least two electrodes, the first switch S1 being arranged in between the power terminal PT and the first electrode terminal E1; a second switch S2 electrically connected to the power terminal PT and a second electrode terminal E2, the second electrode terminal E2 being configured to be electrically connected to another one of said at least two electrodes, the second switch S2 being arranged in between the power terminal PT and the second electrode terminal E2; a third switch S3 electrically connected to the first electrode terminal E1 and a ground terminal GT, the ground terminal GT being configured to be electrically connected to a ground, the third switch S3 being arranged in between the first electrode terminal E1 and the ground terminal GT; and a fourth switch S4 electrically connected to the second electrode terminal E2 and the ground terminal GT, the fourth switch S4 being arranged in between the second electrode terminal E2 and the ground terminal GT. In this connection, switch settings as described above (e.g. with reference to Tables 1 and 2) may be used e.g. for achieving the stimulation signal between the electrodes (e.g. E1 and E2). It should be understood, however, that the medical device 100 according to the present embodiment and used to influence the breathing or a breathing condition of the patient P may be embodied using different control processes and modules. Specifically, the signals applied to the electrode patch 100EP may be generated using a different circuit configuration.

It is advantageous, that the current stimulation of the nervus hypoglossus and/or the musculus genioglossus to influence the breathing condition of the patient P is possible also when the patient P is asleep and specifically the current stimulation can be set such as not to awake the patient P. Furthermore, since the stimulation is done only on the occurrence of the abnormal breathing condition (e.g. in case of an actual or predicted apnea), the potential nuisance to the patient P is reduced to a great extent. The above described medical device 100 for Influencing the breathing condition of the patient P has the advantage that it can be applied without the need of a chirurgic operation. Thus, the medical device 100 can easily applied also just for test purposes, so that patients P are more likely to test the medical device 100. Furthermore, since the stimuli applied to the patient P are electric, a potential/real abnormal breathing condition can be avoided without acoustically impairing the patient P and his surroundings. The medical device 100 can be considered a "passive system" which becomes active only in case of need, i.e. when a (potentially) abnormal breathing condition is detected or predicted. Moreover, the medical device 100 can be used to determine or optimize a location of electrodes of an implanted neurostimulator-system. Furthermore, the medical device 100 can be configured as a portable device and/or can be used for monitoring (e.g. on a long-time basis) the patient's parameters, e.g. vital parameters such as the pulse-transit-time (PTT), the blood pressure, the hearth frequency, electromyogram (EMG), electroencephalogram (EEG), impedance around the trachea, electrocardiogram (ECG, EKG), the electrodermal response (EDR), the pulse amplitude and/or the breathing frequency of the patient P.

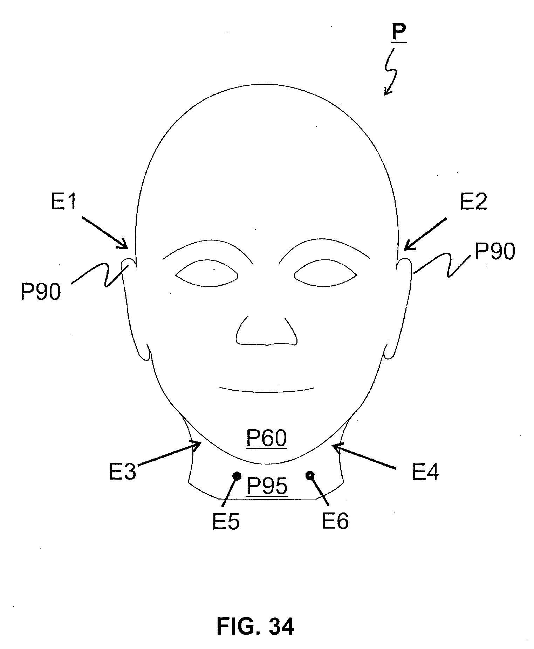

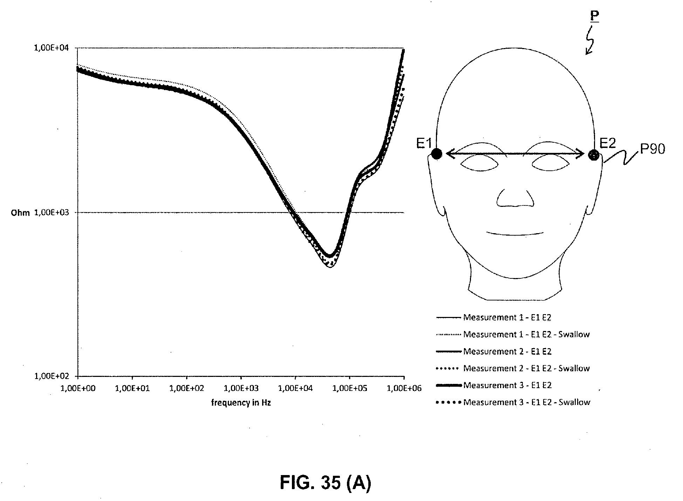

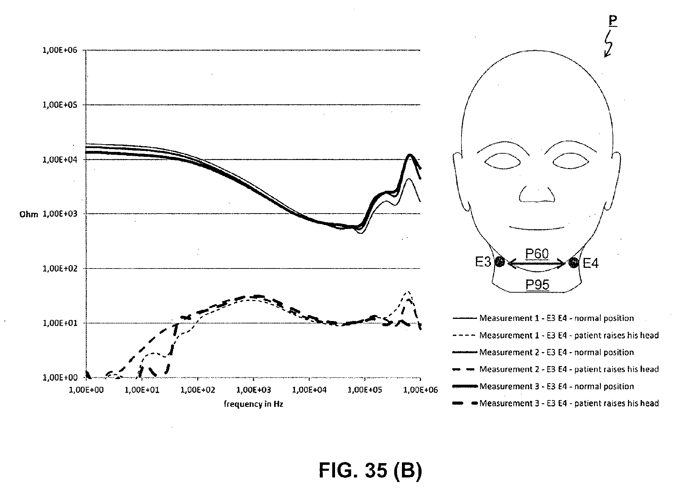

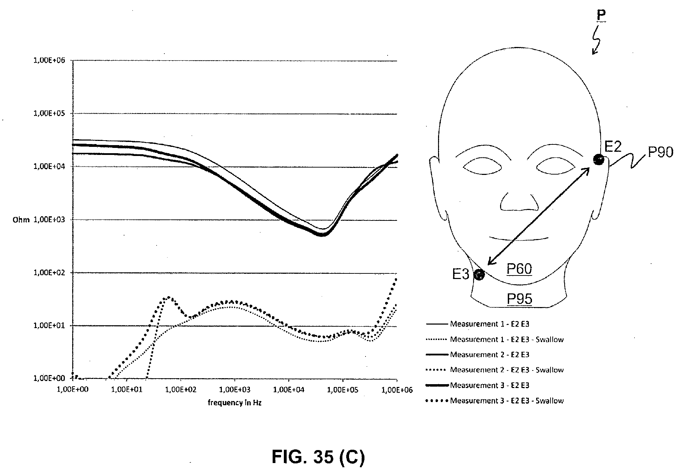

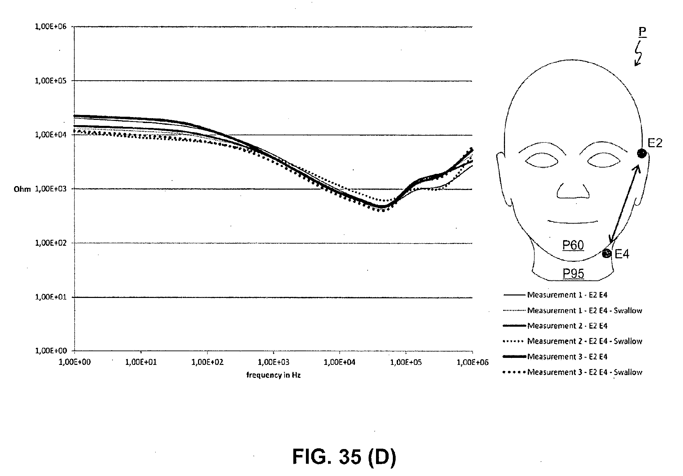

A specified (predetermined or predeterminable) arrangement of electrodes E1 to E6 which may be at least partly arranged on at least one electrode patch 100EP on the patient P is described in reference to **Fig. 34.** The electrodes E1 to E6 are to be placed (directly) on the skin of the patient P on or in proximity of the patient's head. Particularly, a pair of electrodes E1, E2 is to be placed or positioned on or in proximity of the patient's ears P90, specifically beneath and/or behind respective right and left earlobes. One further pair of electrodes E3, E4 are positioned on an area of the patient's neck or throat P95 approximately under the patient's chin P60, specifically substantially between the mandible and the larynx. Still a further pair of electrodes E5, E6 is to be placed or positioned a specified (predetermined or predeterminable) position below the pair of electrodes E3, E4 such as about 2 to 3 cm below on the neck or throat P95. Specifically, electrodes E3 to E6 may be carried on a single electrode patch 100EP (not shown) or may be separately provided. Furthermore, it should be understood that while in Fig. 34 the arrangement of 6 electrodes E1 to E6 is shown, there may be less electrodes arranged, so that 2, 3, 4, 5 or 7 or more electrodes may be arranged (see e.g. the electrode pairs as shown in connection with Fig. 35).

**Fig. 35** shows exemplary signal patterns reflecting frequency dependent resistance measurements between different pairs of electrodes particularly arranged according to Fig. 34.

Specifically, **Fig. 35 (A)** shows the frequency dependent impedance or resistance as measured between two electrodes E1 and E2 arranged on or close to the earlobes P90 of the patient P, specifically beneath the right and left earlobes P90. Since the electrodes E1 and E2 are particularly arranged next to the patient's skull, it is possible to measure the patient's electrical activity of the brain (electroencephalogram, EEG). Specifically, based on the EEG it is possible to monitor and/or detect the sleep activity of the patient P such as sleep depth and/or which sleep stage the patient P is in. In Fig. 35 (A) the solid lines correspond to a normal situation of the patient P while the dotted lines correspond to a situation in which the patient P swallows.

Moreover, the electrodes E1 and E2 as arranged according to Fig. 34 and 35(A) may be used for transcranial DC stimulation of the patient P, which is a common application in neurology and may be used also in connection with treatment and/or detection of sleep apnea.

Furthermore, **Fig. 35 (B)** shows the frequency dependent impedance or resistance as measured between two electrodes E3 and E4 arranged on an area of the patient's neck or throat P95 close to the chin P60 of the patient P, specifically symmetric below the patient's chin P60 on left and right sides and/or below the mandible and/or between the mandible and the larynx. The plain lines shown in the graph correspond to the frequency dependent impedance or resistance as measured in a normal situation of the patient P, while the dashed lines reflect measurements of the impedance or resistance when the patient P raises his/her head.

Furthermore, **Fig. 35 (C)** shows the frequency dependent impedance or resistance as measured between the electrode E2 arranged on or close to the earlobes P90 of the patient P, specifically beneath the right and left earlobes P90 and the electrode E3 arranged on an area of the patient's neck or throat P95 close to the chin P60 of the patient P, specifically below the patient's chin P60 on the side of the patient P opposite to the side, where the electrode E2 is arranged, and/or below the mandible and/or between the mandible and the larynx. The plain lines shown in the graph correspond to the frequency dependent impedance or resistance as measured in a normal situation of the patient P, while the dotted lines reflect measurements of the impedance or resistance when the patient P swallows.

Furthermore, **Fig. 35 (D)** shows the frequency dependent impedance or resistance as measured between the electrode E2 arranged on or close to the earlobes P90 of the patient P, specifically beneath the right and left earlobes P90 and the electrode E4 arranged on an area of the patient's neck or throat P95 close to the chin P60 of the patient P, specifically below the patient's chin P60 on the side of the electrode E2 and/or below the mandible and/or between the mandible and the larynx. The plain lines shown in the graph correspond to the frequency dependent impedance or resistance as measured in a normal situation of the patient P, while the dotted lines reflect measurements of the impedance or resistance when the patient P swallows.

From the graphs of Figs. 35 (A) to 35 (D), it is apparent that by specifically measuring the frequency dependent resistance or impedance between specific pairs of electrodes arranged on the head of the patient P it is possible to draw conclusions on his activity, particularly of his sleep activity (such as whether a situation of sleep apnea occurs.

Moreover, any two of the electrodes E3 to E6 may be used to measure the electromyogram (EMG) of the musculus genioglossus of the patient P, wherein the EMG displays the activity of the musculus genioglossus. As explained, in case of a tracheal obstruction (such as in case of the sleep apnea, specifically hypopnea or apnea) the musculus genioglossus relaxes and cannot keep the tongue P30 in its normal position anymore, so that due to backsliding of the tongue P30 the airway P10 (such as the pharynx or the trachea) is partially or completely closed off, thereby increasing the flow resistance within the airway P10. If the negative pressure inside the lung is not strong enough, the respiratory flow rate will be reduced.

In addition or alternatively to the EMG measurement, any two of the electrodes E3 to E6 may be used to measure an electrodermal activity (EDA). Since the EDA signals are arise from the exocrine glands, which are controlled by nervus sympathicus, it provides valuable hints on a developing sleep apnea or a manifest sleep apnea, since due to the respiratory obstruction an arousal occurs initiated by the central nervous system which can be detected or determined by EDA.

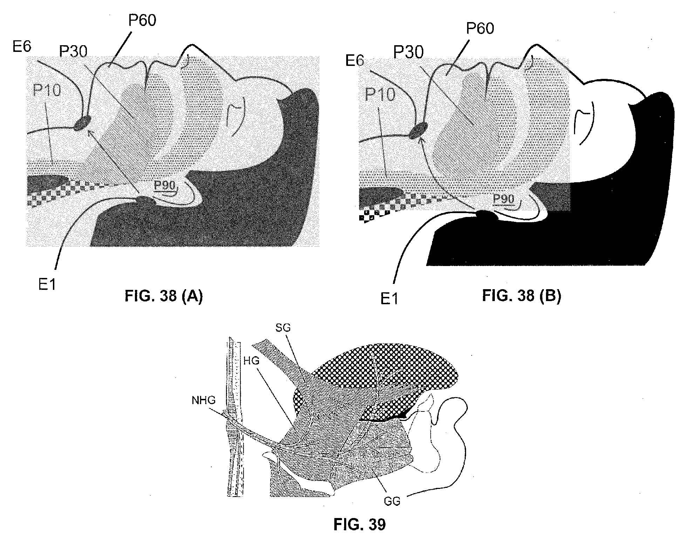

In addition or alternatively to the above measurement(s), pairs of electrodes E1, E4 and/or E1, E6 and/or E2, E3 and/or E2, E5 may be used for electrical impedance tomography (EIT) measurement by applying a low electrical current between the respective pairs of electrodes E1/E4, E1/E6, E2/E3 and/or E2/E5 (electrodes E1 and E6 in the example shown in Fig. 38 (A) and 38 (B)) and measuring the impedance. In other words, by measuring the impedance between a pair of electrodes E1/E4, E1/E6, E2/E3 and/or E2/E5 one being arranged on or near the patient's ear P90 and the other being arranged on the substantially opposite side on or below the patient's chin P60 (also referred to as pairs of electrodes arranged in a crossed pattern or crossed electrodes), an EIT measurement can be performed, wherein the impedance is inversely correlated with the flow resistance within the airway. Specifically, when the patient's airway P10 is open (Fig. 38 (B)) the impedance is high-ohmic, since all currents (see arrow in Fig. 38 (B)) avoid the empty space within the pharynx, so that as a result the respiratory impedance is low-ohmic. On the other side, when the airway P10 is (at least partially) obstructed (see Fig. 38 (A), Fig. 31 (B-1) and (B-2)), e.g. as caused by a backsliding tongue P30, the tongue P30 serves as a short circuit for the currents between the pairs of electrodes E1/E4, E1/E6, E2/E3 and/or E2/E5 (see Fig. 38 (A)), so that as a result the respiratory impedance is very high-ohmic. Accordingly, electrical impedance measurements can be used to diagnose breathing and the respiratory impedance, giving valuable information about the degree of obstruction (see measurements according to Figs. 35 (A) to 35(D) and Figs. 38 (A) and 38 (B)).

In addition or alternatively to the above measurement(s), pairs of electrodes E1/E4, E1/E6, E2/E3 and/or E2/E5 may be used for an electrocardiogram (ECG) measurement. Specifically, the pairs of electrodes E1/E4, E1/E6, E2/E3 and/or E2/E5 arranged in a crossed pattern may be used also without application of an external electrical current thereto. In fact, since the crossed electrodes E1/E4, E1/E6, E2/E3 and/or E2/E5 are not positioned on an equipotential line of the heart dipole, the voltages across these crossed electrodes E1/E4, E1/E6, E2/E3 and E2/E5 match or correspond to (or are an indicator of) the voltages in an electrocardiogram (ECG). Specifically, during the diagnostics of sleep apnea an ECG is commonly recorded.

In addition or alternatively to the above measurement(s), any two or more of the electrodes E3 to E6 may be used for transcutaneous stimulation, wherein an electrical current is applied to any two (or more) of the four electrodes E3 to E6 for transcutaneous stimulation. Specifically, by selecting a suitable stimulation pattern the nervus hypoglossus and the musculus genioglossus (GG) can be stimulated (Fig. 39), whereby the tongue P30 can be moved substantially forward so that the flow resistance within the airway P10 can be strongly decreased. Specifically, the muscles M. hyoglossus (HG) and M. styloglossus (SG) serve as the antagonists of the M. genioglossus (GG) **(Fig. 39).** During a balance of forces the tongue P30 substantially is kept in its normal position, and the airway P10 is open. During a relaxation of GG the tongue P30 substantially slides backward due to tensile forces of HG and SG. Accordingly, a favorable balance of forces can be obtained by stimulating for example electrodes E3/E5 and/or E4/E6 (and/or diagonally electrodes E3/E6 and/or E4/E5), wherein "favorable" particularly refers to a substantially obstruction-free respiratory flow rate, which is soft and not limited.

Accordingly, the positioning of electrodes E1 to E6 as described above and the accrued benefits enable diagnosis and treatment of an obstructive sleep apnea (OSA). Moreover, the electrical impedance tomography (EIT) may be based on the measurement of electrical impedances around an object with varying impedance elements. For example, it is possible to perform EIT of the patient's lung e.g. directly at the patient's bed, unlike computer tomography (CT) or nuclear magnetic resonance (NMRI). When the objects of interest are the trachea, the pharynx and/or the esophagus, specifically high-ohmic impedances can be detected between specific (predetermined or predeterminable) pairs of electrodes of the plurality of electrodes E1 to E6. Particularly, when positioning the plural electrodes E1 to E6 as described above and/or with (specifically additional) electrodes around throat and/or neck any movements during swallowing can be detected particularly with the device as described with reference to Figs. 1 to 30.

**Claims**

1. A medical device (10) comprising a first circuit (12, C0) for selectively applying power to at least two electrodes applied to a human or animal subject, the first circuit (12, C0) including:

   a first switch (S1) electrically connected to a power terminal (PT) and a first electrode terminal (E1), the power terminal (PT) being configured to receive input power from a power source, the first electrode terminal (E1) being configured to be electrically connected to one of said at least two electrodes, the first switch (S1) being arranged in between the power terminal (PT) and the first electrode terminal (E1);
   a second switch (S2) electrically connected to the power terminal (PT) and a second electrode terminal (E2), the second electrode terminal (E2) being configured to be electrically connected to another one of said at least two electrodes, the second switch (S2) being arranged in between the power terminal (PT) and the second electrode terminal (E2);
   a third switch (S3) electrically connected to the first electrode terminal (E1) and a ground terminal (GT), the ground terminal (GT) being configured to be electrically connected to a ground, the third switch (S3) being arranged in between the first electrode terminal (E1) and the ground terminal (GT); and
   a fourth switch (S4) electrically connected to the second electrode terminal (E2) and the ground terminal (GT), the fourth switch (S4) being arranged in between the second electrode terminal (E2) and the ground terminal (GT).

**2.** The medical device (10) according to claim 1, wherein:

the first circuit (12, C0) further comprises a ground connecting switch (SG) that is electrically connected to the ground terminal (GT) and that is configured to electrically connect or disconnect the ground terminal (GT) to the ground; and
the ground connecting switch (SG) may be implemented by two switches (SG', SG") electrically connected in parallel to the ground terminal (GT).

**3.** The medical device (10) according to claim 1 or 2, wherein:

the first circuit (12, C0) further comprises a first differential amplifier (A1), a second differential amplifier (A2), a first measurement terminal (M1), a second measurement terminal (M2), a fifth switch (S5), a sixth switch (S6) and a resistance ($R_M$);
each of the first measurement terminal (M1) and the second measurement terminal (M2) is electrically connected to one of two input terminals of the first differential amplifier (A1);
a first end of the third switch (S3) is electrically connected to the first electrode terminal (E1) and a second end of the third switch (S3) is electrically connected to the ground terminal (GT) via the fifth switch (S5) and the resistance ($R_M$);
the second end of the third switch (S3) is further electrically connected to the first measurement terminal (M1);
a first end of the fourth switch (S4) is electrically connected to the second electrode terminal (E2) and a second end of the fourth switch (S4) is electrically connected to the ground terminal (GT) via the sixth switch (S6) and the resistance ($R_M$);
the second end of the fourth switch (S4) is further electrically connected to the second measurement terminal (M2);
a first end of the fifth switch (S5) is electrically connected to the second end of the third switch (S3) and to the first measurement terminal (M1);
a second end of the fifth switch (S5) is electrically connected to a first end of the resistance ($R_M$);
a first end of the sixth switch (S6) is electrically connected to the second end of the fourth switch (S4) and to the second measurement terminal (M2);
a second end of the sixth switch (S6) is electrically connected to the first end of the resistance ($R_M$);
a second end of the resistance ($R_M$) is electrically connected to the ground terminal (GT); and
each of the first and second ends of the resistance ($R_M$) is electrically connected to one of two input terminals of the second differential amplifier (A2).

**4.** The medical device (10) according to claim 1 or 2, wherein:

the first circuit (12, C0) further comprises a first differential amplifier (A1), a second differential amplifier (A2), a circuit breaker (CB), a fifth switch (S5), a sixth switch (S6) and a resistance ($R_M$);
each of the first and second electrode terminals (E1, E2) is electrically connected to one of two input terminals of the first differential amplifier (A1) via the circuit breaker (CB), the circuit breaker (CB) being configured to electrically disconnect the first differential amplifier (A1) from the first and second electrode terminals (E1, E2) in case a voltage applied between the first and second electrode terminals (E1, E2) is higher than a specified threshold value;
a first end of the third switch (S3) is electrically connected to the first electrode terminal (E1) and a second end of the third switch (S3) is electrically connected to the ground terminal (GT) via the fifth switch (S5) and the resistance ($R_M$);
a first end of the fourth switch (S4) is electrically connected to the second electrode terminal (E2) and a second end of the fourth switch (S4) is electrically connected to the ground terminal (GT) via the sixth switch (S6) and the resistance ($R_M$);
the second end of the third switch (S3) and the second end of the fourth switch (S4) are electrically connected to each other;
a first end of the fifth switch (S5) is electrically connected to the second end of the third switch (S3) and a second end of the fifth switch (S5) is electrically connected to a first end of the resistance ($R_M$);
a first end of the sixth switch (S6) is electrically connected to the second end of the fourth switch (S4) and a second end of the sixth switch (S6) is electrically connected to the first end of the resistance ($R_M$);
a second end of the resistance ($R_M$) is electrically connected to the ground terminal (GT); and
each of the first and second ends of the resistance ($R_M$) is electrically connected to one of two input terminals of the second differential amplifier (A2).

5. The medical device (10) according to any one of the preceding claims, further comprising at least one second circuit (12, C1), wherein:

said at least one second circuit (12, C1) has the features of the first circuit (12, C0);
the first and second electrode terminals (E3, E4) of said at least one second circuit (12, C1) are configured to be electrically connected to electrodes that are different from the electrodes to be connected to the first and second electrode terminals (E1, E2) of the first circuit (12, C0);
the first circuit (12, C0) and said at least one second circuit (12, C1) may be configured to receive input power from different power sources; and
the first circuit (12, C0) and said at least one second circuit (12, C1) may be galvanically isolated from one another either by true galvanic isolation or virtual galvanic isolation.

6. A method performed by a controller (20) configured to control the medical device (10) according to any one claims 1 to 4, the method comprising controlling the medical device (10) to operate, in accordance with a setting made by a user, in one of the following modes:

a stimulation mode in which a part of the human or animal subject is electrically stimulated by two electrodes connected to the first and second electrode terminals (E1, E2), wherein the controller (20) controls the medical device (10) to set:

the first and fourth switches (S1, S4) to a closed state and the second and third switches (S2, S3) to an open state; or
the first and fourth switches (S1, S4) to the open state and the second and third switches (S2, S3) to the closed state;

an inactive mode wherein the controller (20) controls the medical device (10) to set the first, second, third and fourth switches (S1, S2, S3, S4) to the open state;
a short circuit mode wherein the controller (20) controls the medical device (10) to set the first and second switches (S1, S2) to the open state and the third and fourth switches (S3, S4) to the closed state.

7. The method according to claim 6, wherein:

the controller (20) is configured to control the medical device (10) of claim 2;
in the stimulation mode and the inactive mode, the controller (20) controls the medical device (10) to set the ground connecting switch (SG) to the closed state; and
in the short circuit mode, the controller (20) controls the medical device (10) to set the ground connecting switch (SG) to the open state.

8. The method according to claim 6 or 7, wherein:

the controller (20) is configured to control the medical device (10) of claim 3;
in the inactive mode, the controller (20) controls the medical device (10) to set the fifth and sixth switches (S5, S6) to the closed state while setting the first, second, third and fourth switches (S1, S2, S3, S4) to the open state;
in the stimulation mode, the controller (20) controls the medical device (10) to set the fifth and sixth switches (S5, S6) to the closed state;
in the short circuit mode, the controller (20) controls the medical device (10) to set the first and second switches (S1, S2) to the open state and the third, fourth, fifth and sixth switches (S3, S4, S5, S6) to the closed state;
the controller (20) is further configured to control the medical device (10) to operate, in accordance with the setting by the user, in the stimulation mode, the inactive mode, the short circuit mode, a signal measurement mode or an impedance measurement mode, wherein
in the signal measurement mode, the controller (20) controls the medical device (10) to:

set the first, second, fifth and sixth switches (S1, S2, S5, S6) to the open state and the third and fourth switches (S3, S4) to the closed state; and
determine a signal between the first and second electrode terminals (E1, E2) using an output from the first differential amplifier (A1); and

in the impedance measurement mode, the controller (20) controls the medical device (10) to:

set the third and fourth switches (S3, S4) to the closed state while setting:

the first and sixth switches (S1, S6) to the closed state and the second and fifth switches (S2, S5) to the open state; or
the first and sixth switches (S1, S6) to the open state and the second and fifth switches (S2, S5) to the closed state; and

determine an impedance between the first and second electrode terminals (E1, E2) using outputs from the first and second differential amplifiers (A1, A2).

9. The method according to claim 6 or 7, wherein:

the controller (20) is configured to control the medical device (10) of claim 4;
in the stimulation mode, the controller (20) controls the medical device (10) to:

set one or both of the fifth and sixth switches (S5, S6) to the closed state; and
set the circuit breaker (CB) to the open state;

in the inactive mode, the controller (20) controls the medical device (10) to set the fifth and sixth switches (S5, S6) to the closed state and the circuit breaker (CB) to the open state;
in the short circuit mode, the controller (20) controls the medical device (10) to set the fifth and sixth switches (S5, S6) to the open state and the circuit breaker (CB) to the open state; and
the controller (20) is configured to control the medical device (10) to operate, in accordance with the setting by the user, in the stimulation mode, the inactive mode, the short circuit mode or a signal measurement mode, wherein, in the signal measurement mode, the controller controls the medical device to:

set the first, second, third and fourth switches (S1, S2, S3, S4) to the open state;
set the fifth and sixth switches (S5, S6) to the closed state;
set the circuit breaker (CB) to the closed state; and
determine a signal between the first and second electrode terminals (E1, E2) using an output from the first differential amplifier (A1).

10. A method performed by a controller (20) configured to control the medical device (10) according to claim 5, the method comprising:

controlling the medical device (10) to operate in a stimulation mode in case of electrically stimulating a part of the human or animal subject by a pair of electrodes connected to a pair of electrode terminals selected from the first and second electrode terminals (E1, E2, E3, E4) in the first and second circuits (12, C0, C1), wherein, in the stimulation mode, the controller (20) controls the medical device (10) to set each switch in the first and second circuits (12, C0, C1) to the closed state or the open state so as to:

electrically connect one of the selected pair of electrode terminals to the power terminal (PT) of the circuit comprising the one of the selected pair of electrode terminals;
electrically connect another one of the selected pair of electrode terminals to the ground terminal (GT) of the circuit comprising the other one of the selected pair of electrode terminals; and
electrically disconnect the first and second electrode terminals other than the selected pair of electrode terminals from the ground terminal (GT) and from the power terminal (PT).

11. The method according to claim 10, wherein:

the controller (20) is configured to control the medical device (10) having the features of claims 3 and 5;
in the stimulation mode, the controller (20) controls the medical device to set the fifth and sixth switches (S5, S6) of the first and second circuits (12, C0, C1) to the closed state;
the controller (20) is further configured to control the medical device (10) to operate in an impedance measurement mode in case of measuring an impedance between a pair of electrode terminals selected from the first and second electrode terminals (E1, E2, E3, E4) in the first and second circuits (12, C0, C1), wherein, in the impedance measurement mode, the controller (20) controls the medical device (10) to:

when the selected pair of electrode terminals are comprised in a same circuit,

set the third and fourth switches (S3, S4) of that circuit to the closed state while setting:

the first and sixth switches (S1, S6) of that circuit to the closed state and the second and fifth switches (S2, S5) of that circuit to the open state; or
the first and sixth switches (S1, S6) of that circuit to the open state and the second and fifth switches (S2, S5) of that circuit to the closed state; and

set the first and second switches (S1, S2) of the first or second circuit that does not comprise the selected pair of electrode terminals to the open state; and

when each one of the selected pair of electrode terminals are comprised in a different circuit, set each switch of the first and second circuits (12, C0, C1) to the closed state or the open state so as to:

electrically connect one of the selected pair of electrode terminals to the power terminal (PT) and the first or second measurement terminal of the circuit comprising the one of the selected pair of electrode terminals;
electrically connect, to the ground terminal (GT) via the resistance ($R_M$), the first or second measurement terminal that is comprised in the circuit comprising the one of the selected pair of electrode terminals and that is not electrically connected to the one of the selected pair of electrode terminals;
electrically connect another one of the selected pair of electrode terminals to the first or second measurement terminal of the circuit comprising the other one of the selected pair of electrode terminals;
electrically connect, to the ground terminal (GT) via the resistance ($R_M$), the first and second measurement terminals of the circuit comprising the other one of the selected pair of electrode terminals; and
electrically disconnect the first and second electrode terminals other than the selected pair of electrode terminals from the ground terminal (GT) and from the power terminal (PT).

12. A computer program product comprising computer-readable instructions that, when loaded and run on a computer, cause the computer to perform the method according to any one of claims 6 to 11.

13. A controller (20) configured to perform the method according to any one of claims 6 to 11.

14. A system (1) comprising:

the medical device (10) according to any one of claims 1 to 5; and
the controller (20) according to claim 13.

15. The system (1) according to claim 14, further comprising:

a floating current source as the power source that supplies the input power to the power terminal (PT) of the medical device (10).

**FIG. 1**

<u>1</u>

20

Controller

System Memory
240 — RAM    ROM    242 — 24

22 — Processing Unit

30

34a    34b    34c    26    30

I/O    I/O    I/O    HDD Interface    External Disk Drive Interface

36 — Input Device    Display Device — 38

HDD    External Disk Drive
28 — HDD    32 — External Disk Drive

Medical Device

10 —

12 — Circuit    Electrode 1    50-1

Electrode 2    50-2

⋮

FIG. 2

**FIG. 3**

**FIG. 4**

$V_H$

C0

$i_{pattern1}$

PT0

S01    S02

E1    E2

S03    S04

GT0

C1

$i_{pattern2}$

PT1

S11    S12

E3    E4

S13    S14

GT1

• • •

GND

**FIG. 5**

FIG. 6

FIG. 7

FIG. 8

--- Envelope curve A produced by current source

········· Envelope curve B produced by switching switch-settings

▧ Actual form for stimulation Ch 1

**FIG. 9**

FIG. 10

**FIG. 11**

FIG. 12

**FIG. 13**

**FIG. 14**

FIG. 15

**FIG. 16**

**FIG. 17**

FIG. 18

FIG. 19

FIG. 20

$$u_{a1} = A1 \cdot u_{M2-M1} = A1 \cdot \Delta u_E$$

$$u_{a2} = A2 \cdot u_M = A2 \cdot u_{Sens}$$

**FIG. 21**

**FIG. 22**

FIG. 23

FIG. 24

FIG. 25A

$u_{a1} = A1 \cdot u_{M2-M1} = A1 \cdot \Delta u_E$

$A2 \cdot u_M = A2 \cdot u_{Sens}$

**FIG. 25B**

FIG. 26A

FIG. 26B

**FIG. 27A**

**FIG. 27B**

FIG. 28A

**FIG. 28B**

**FIG. 29**

FIG. 30

(A-1)  (B-1)  (C-1)

P
P40  P50
P10
P30
N  P20
B-N
normal

P
P40  P50
P10
P30
R  P20
B-R
hypopnea

P
P40  P50
P10
P30
O  P20
B-O
apnea

(A-2)  (B-2)  (C-2)

FIG. 31

**FIG. 32**

**FIG. 33**

**FIG. 34**

FIG. 35 (A)

FIG. 35 (B)

FIG. 35 (C)

FIG. 35 (D)

**FIG. 36**

FIG. 37

**FIG. 38 (A)**

**FIG. 38 (B)**

**FIG. 39**

Europäisches
Patentamt

European
Patent Office

Office européen
des brevets

# EUROPEAN SEARCH REPORT

Application Number

EP 15 00 3569

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/239112 A1 (MURAOKA YOSHIHIRO [JP]) 20 September 2012 (2012-09-20) * abstract; figures 1-7 * * paragraphs [0029] - [0032], [0046] - [0047], [0059] - [0068], [0083] - [0091] * | 1-15 | INV. A61B5/00 A61B5/0205 A61B5/08 A61N1/00 A61N1/04 A61N1/36 |
| X | US 2014/343625 A1 (Ó LAIGHIN GEARÓID [IE] ET AL) 20 November 2014 (2014-11-20) * abstract; figures 1-9 * * paragraphs [0053] - [0061], [0078] - [0084] * | 1-15 | |
| X A | US 2015/327808 A1 (VICE JACK M [US]) 19 November 2015 (2015-11-19) * abstract; figures 1-8 * * paragraphs [0002] - [0007], [0027] - [0046] * | 1,6,12, 13 2-5, 7-11,14, 15 | |
| A | US 2015/223710 A1 (CONG PENG [CN] ET AL) 13 August 2015 (2015-08-13) * abstract; figures 1-8 * * paragraphs [0006] - [0009], [0018] - [0046] * | 1-15 | TECHNICAL FIELDS SEARCHED (IPC) A61B A61N H04Q |
| A | US 4 300 566 A (STINDT RICHARD E ET AL) 17 November 1981 (1981-11-17) * figure 1 * * column 2, lines 20-56 * * column 3, line 10 - column 8, line 55 * | 1-15 | |

The present search report has been drawn up for all claims

| Place of search | Date of completion of the search | Examiner |
|---|---|---|
| The Hague | 12 May 2016 | Carta, Riccardo |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 00 3569

This annex lists the patent family members relating to the patent documents cited in the above-mentioned European search report.
The members are as contained in the European Patent Office EDP file on
The European Patent Office is in no way liable for these particulars which are merely given for the purpose of information.

12-05-2016

| Patent document cited in search report | | Publication date | Patent family member(s) | | Publication date |
|---|---|---|---|---|---|
| US 2012239112 | A1 | 20-09-2012 | CN 102711906 A | | 03-10-2012 |
| | | | EP 2497529 A1 | | 12-09-2012 |
| | | | JP 5725562 B2 | | 27-05-2015 |
| | | | US 2012239112 A1 | | 20-09-2012 |
| | | | WO 2011055518 A1 | | 12-05-2011 |
| US 2014343625 | A1 | 20-11-2014 | EP 2776122 A1 | | 17-09-2014 |
| | | | US 2014343625 A1 | | 20-11-2014 |
| | | | WO 2013069003 A1 | | 16-05-2013 |
| US 2015327808 | A1 | 19-11-2015 | US 2015327808 A1 | | 19-11-2015 |
| | | | WO 2015179379 A1 | | 26-11-2015 |
| US 2015223710 | A1 | 13-08-2015 | NONE | | |
| US 4300566 | A | 17-11-1981 | NONE | | |

EPO FORM P0459

For more details about this annex : see Official Journal of the European Patent Office, No. 12/82